# EUROPEAN PATENT SPECIFICATION

(11) **EP 1 694 184 B1**
(45) Date of publication and mention of the grant of the patent: **28.10.2009**
(21) Application number: 04813835.8
(22) Date of filing: 10.12.2004
(51) Int. Cl.: A47K 7/03, A61Q 19/10, A61K 8/63, A61K 8/98

(54) **CHILD S CLEANING IMPLEMENT COMPRISING A BIOLOGICAL EXTRACT**
REINIGUNGSVORRICHTUNG FÜR KINDER MIT EINEM BIOLOGISCHEN EXTRAKT
ACCESSOIRE DE NETTOYAGE POUR ENFANT COMPRENANT UN EXTRAIT BIOLOGIQUE

(30) Priority: 16.12.2003 US 737423
(43) Date of publication of application: 30.08.2006
(73) Proprietor: The Procter and Gamble Company, Cincinnati, Ohio 45202 (US)
(72) Inventor: BENJAMIN, Joyce, Marie, Cincinnati, Ohio 45240 (US); MASON, Michael, Wayne, Cincinnati, Ohio 45249-1651 (US); MASON, Jenna, Lebanon, Ohio 45036 (US)
(74) Representative: Kremer, Véronique Marie Joséphine
(86) International application number: PCT/US2004/041575
(87) International publication number: WO 2005/058241

(56) References cited:
- WO-A-01/08641
- WO-A-03/000106
- WO-A-2004/080256
- WO-A-2004/080257
- WO-A-2004/080258
- FR-A- 2 813 777
- US-A1- 2002 177 535
- US-B1- 6 292 949

## Description

### FIELD OF INVENTION

A disposable child sized cleaning implement is provided. The disposable child sized cleaning implement is releasably carrying a personal care composition which comprises a biological extract.

### BACKGROUND OF THE INVENTION

The skin is naturally an excellent barrier to the penetration of many foreign substances. From time-to-time, the natural ability of the skin to protect is compromised by external factors including abrasions, irritants and the like. Attempts have been made in recent years to promote skin health through the use of various products containing additives or developing synthetic or naturally occurring polymers that mimic or complement the properties of skin in order to maintain the skin health.

It is known that various agents have skin enhancing properties when applied to skin and hair. Effective delivery of agents that can enhance or prevent damage to the underlying protective barrier of skin, is an interest in a variety of medical as well as personal care industries such as dermatology and cosmetics, respectively.

Additionally, Consumer products, such as, cleansing and conditioning products as well as household consumer cleaning products, have traditionally been marketed in a variety of forms such as bar soaps, creams, foams, sprays, liquids, powders, lotions, and gels. Typically, these products must satisfy a number of criteria to be acceptable to consumers. These criteria include effectiveness, skin feel, mildness to skin, suitability for use in the consumer's household, and appearance. Typically these consumer products comprise a composition in some form.

It is highly desirable to deliver personal care compositions, especially those that care for the skin from a disposable substrate. Disposable products are convenient because they obviate the need to carry or store cumbersome bottles, bars, jars, tubes, and other forms of clutter associated with consumer products. Disposable products are also a more sanitary alternative to the use of a sponge, washcloth, or other cleansing implement intended for extensive reuse, because such implements can develop bacterial growth, unpleasant odors, and other undesirable characteristics related to repeated use, even if advancements have been made to clean and dry them quickly (US 6292949). Disposable personal care articles have been developed such as gloves including a cleansing product absorbed within the layers of the gloves (FR 2813777), articles including a therapeutic benefit component (WO 01/08641) or nonwoven mitts releasably carrying a personal care composition (WO 04/080256, WO 04/080257, WO 04/080258, patent applications made available to the public after the priority date of the present invention).

It can now be appreciated that using consumer products involves many aspects for both the child and the caregiver, especially for the child incapable of reading. Some of these aspects affect children differently, or may not even be a factor for a particular child. It is this uniqueness of each individual child that presents a major challenge for both the child and the caregiver. If any of these aspects are unsuccessful, the child's progress in learning how to, for example bathe or clean properly can be unnecessarily delayed due to numerous failures and frustrations. In the past reusable washcloths and sponges have been made in various shapes, such as puppets and with child appealing graphics, in order to make the use of these products fun and enjoyable. However, these reusable products still suffer from the problems associated with repeated use, i.e. bacterial growth, unpleasant odors, and other undesirable characteristics related to extensive reuse. On the other hand, while disposable products side step this problem of extensive reuse, no effort has been made to make disposable products more appealing to children.

Furthermore, any consumer product which is specifically designed to thoroughly clean a child's skin should remaining gentle enough to preserve the health of a child's, skin. Ideally, any such product would leave the skin feeling soft and supple be able to at least mitigate, if not reverse, any damage done to the child's skin, such as reducing skin hydration and the like.

The problem remains that there is no disposable cleaning articles, products or system available for children of all ages and sizes, which can be easily handled and the method of utilizing easily understood, by the child. The need also remains for disposable cleaning products which are easy to use, suitable for use by and attractive and appealing to children, of different ages, all sizes and/or stages of development and thoroughly clean a child's skin while remaining gentle enough to preserve the health of a child's, skin.

### SUMMARY OF THE INVENTION

A first aspect of the present invention provides, a disposable child sized cleaning implement comprising:
(a) a personal care composition, wherein the personal care composition comprises a biological extract, being selected from the group consisting of:
   (i) a sterol;
   (ii) an animal extract,
      wherein the disposable child sized cleaning implement is releasably carrying the personal care composition; and
(b) child graphic disposed on the disposable cleaning implement. The child graphic appeals to a child such that the child will interact with the implement in the typical use to which the implement is put.

A second aspect, the present invention provides, a disposable nonwoven child's cleansing mitt adapted to fit on a child's hand comprising:
(a) a personal care composition, wherein the personal care composition comprises a biological extract, being selected from the group consisting of:
   (i) a sterol;
   (ii) an animal extract,
   wherein the disposable nonwoven child's cleansing mitt is releasably carrying the personal care composition; and
(b) child graphic disposed on the Disposable nonwoven child's cleansing mitt. The child graphic appeals to a child such that the child will interact with the implement in the typical use to which the implement is put.

A third aspect, the present invention provides, a disposable nonwoven mitt adapted to fit on a child's hand, comprising:
(a) first and second nonwoven sheet members in an overlying relationship, the members defining an interior volume for receiving the child's hand, each of the first and second nonwoven sheet members including an exterior surface, having an opposing interior surface, a top edge, a bottom edge opposing the top edge, and first and second opposed side edges, the first and second nonwoven sheet members being permanently secured to each other along the periphery of the top edge and both of the first and second opposed side edges, with the bottom edges being unsecured so as to provide a substantial access opening to the interior volume for readily inserting the child's hand therein;
(b) a personal care composition, wherein the personal care composition comprises a biological extract, being selected from the group consisting of:
   (i) a sterol;
   (ii) an animal extract,
   wherein at least one of the first and second sheet members is releasably carrying the personal care composition; and
(c) child graphic disposed on at least one of the first and second sheet members. The child graphic appeals to a child such that the child will interact with the implement in the typical use to which the implement is put.

It should be understood that every limit given throughout this specification will include every lower, or higher limit, as the case may be, as if such lower or higher limit was expressly written herein. Every range given throughout this specification will include every narrower range that falls within such broader range, as if such narrower ranges were all expressly written herein. All percentages, ratios and proportions are by weight, and all temperatures are in degrees Celsius (°C), unless otherwise specified. All measurements are in SI units unless otherwise specified.

### BRIEF DESCRIPTION OF THE DRAWINGS

The above-mentioned and other features and objects of this invention and the manner of attaining them will become more apparent, and the invention itself will be better understood by reference to the following description of the invention taken in conjunction with the accompanying drawings, wherein:
Figure 1 illustrates one embodiment of a disposable cleaning implement.
Figure 2 illustrates one embodiment of a disposable nonwoven child's cleansing mitt.
Figure 3 illustrates another alternative embodiment of a disposable nonwoven child's cleansing mitt.
Figure 4 illustrates a child graphic.
Figure 6 illustrates another child graphic.

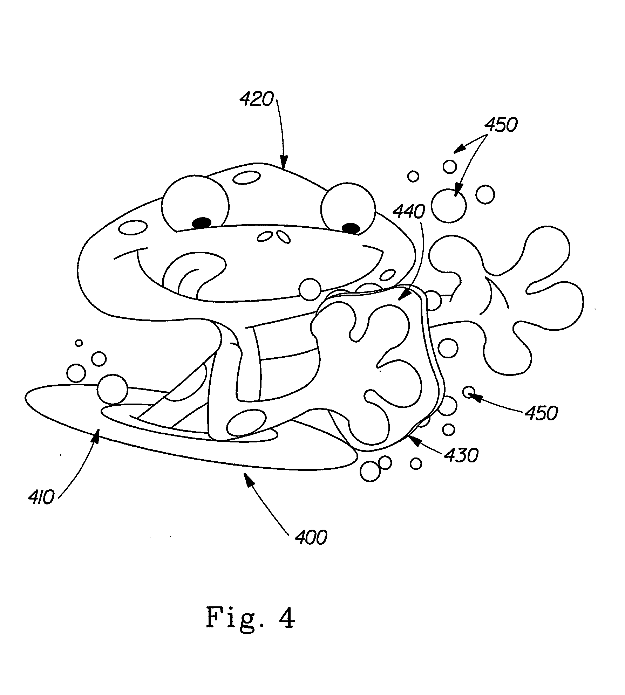

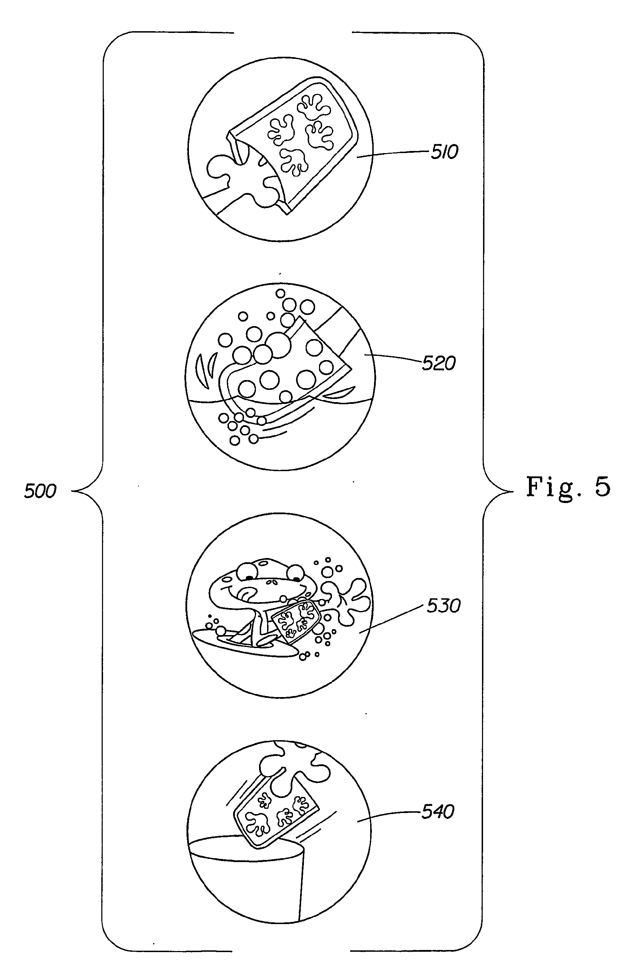

Figure 5 Illustrates one embodiment of a disposable nonwoven child's cleansing mitt having a portion of child graphic of Figure 4.

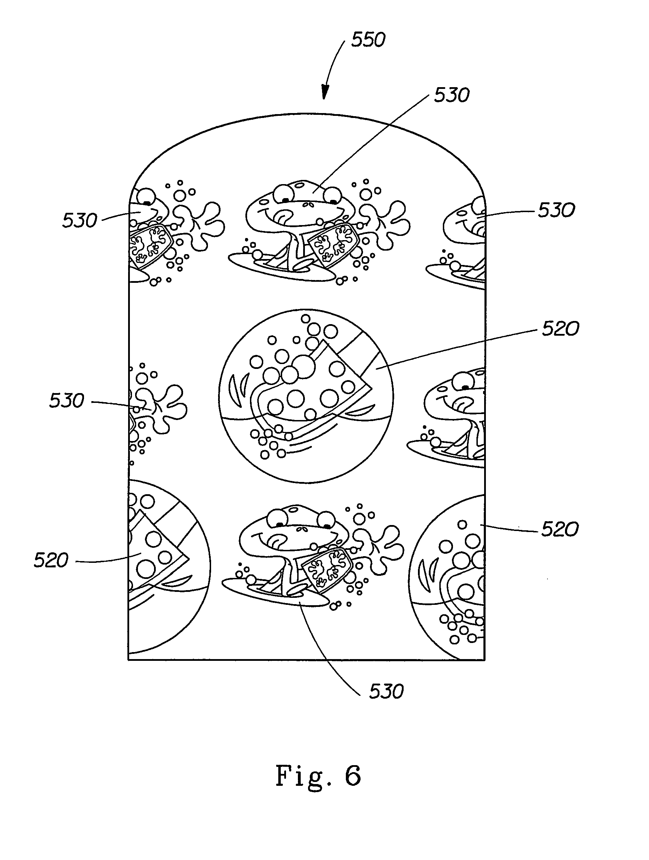

Figure 6 illustrates another child graphic.

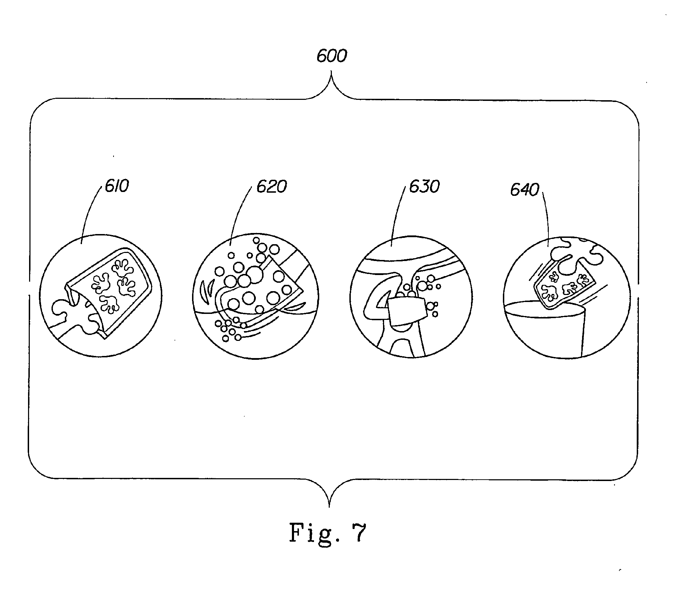

Figure 7 illustrates another child graphic.

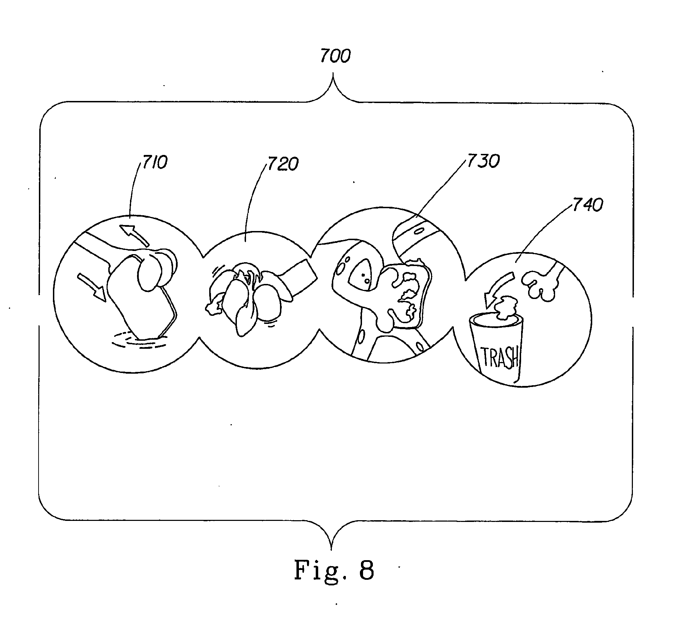

Figure 8 illustrates another child graphic.
Figure 7 illustrates another child graphic.

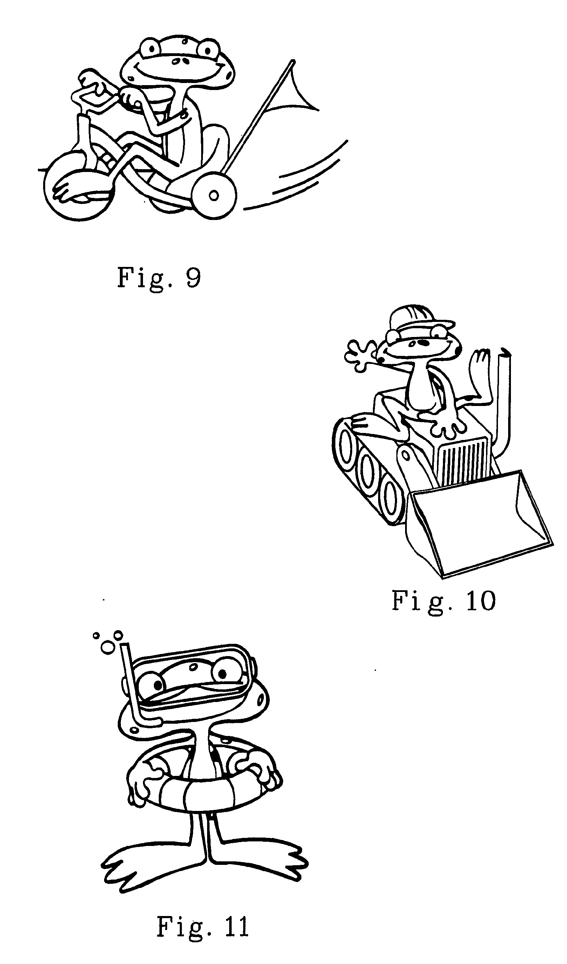

Figure 9 illustrates another child graphic.
Figure 10 illustrates anther child graphic.
Figure 11 illustrates another child graphic.

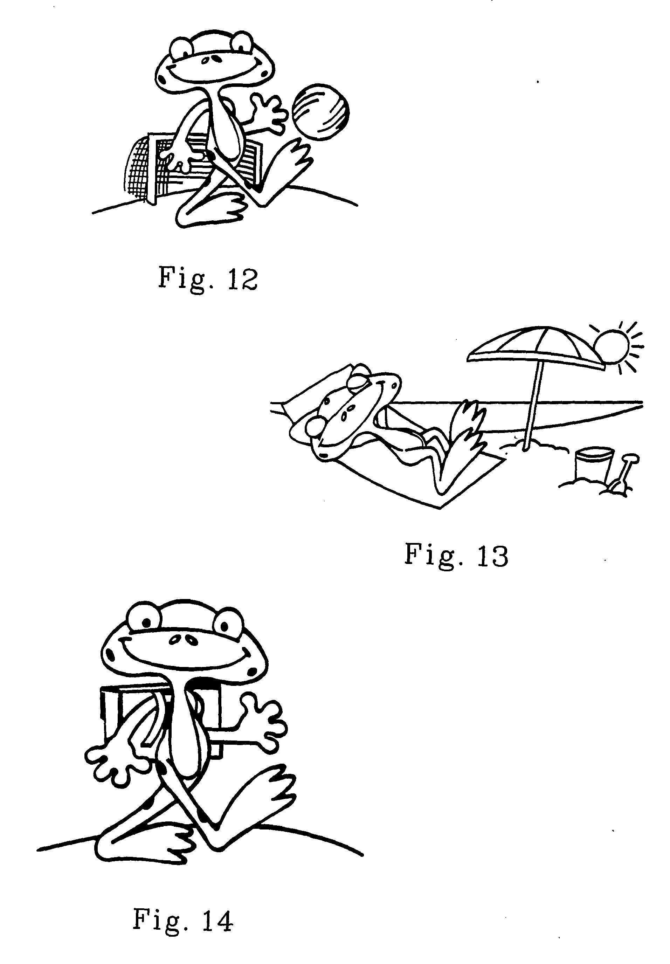

Figure 12 illustrates another child graphic.
Figure 13 illustrates another child graphic.
Figure 14 illustrates another child graphic.

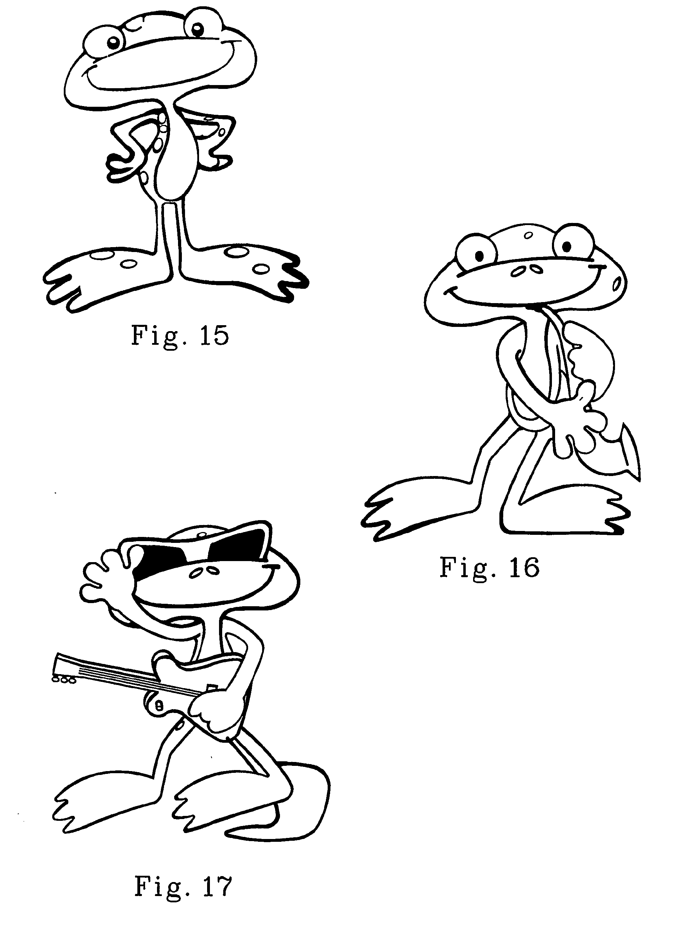

Figure 15 illustrates another child graphic.
Figure 16 illustrates another child graphic.
Figure 17 illustrates another child graphic.

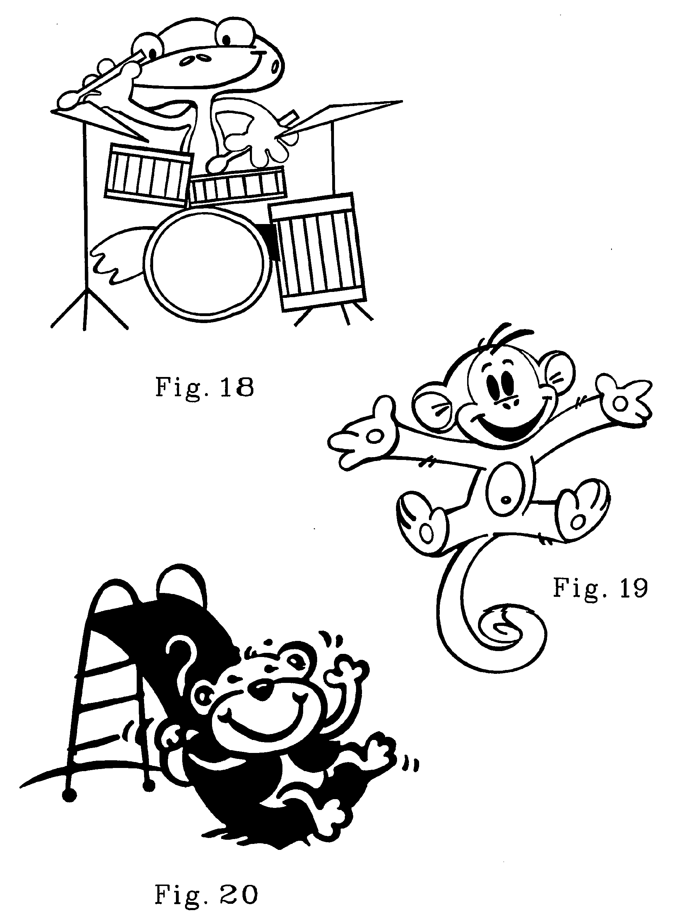

Figure 18 illustrates another child graphic.
Figure 19 illustrates another child graphic.
Figure 20 illustrates another child graphic.

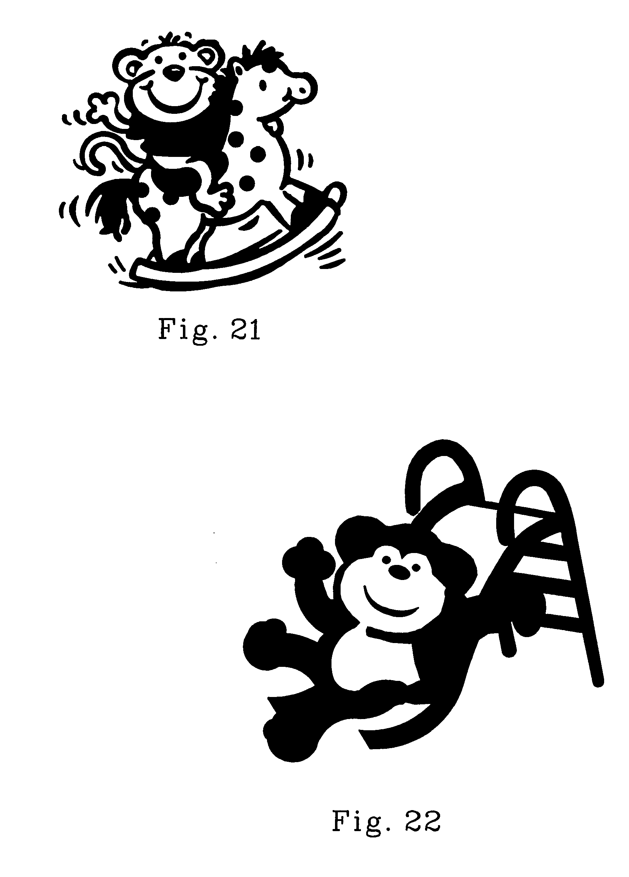

Figure 21 illustrates another child graphic.
Figure 22 illustrates another child graphic.

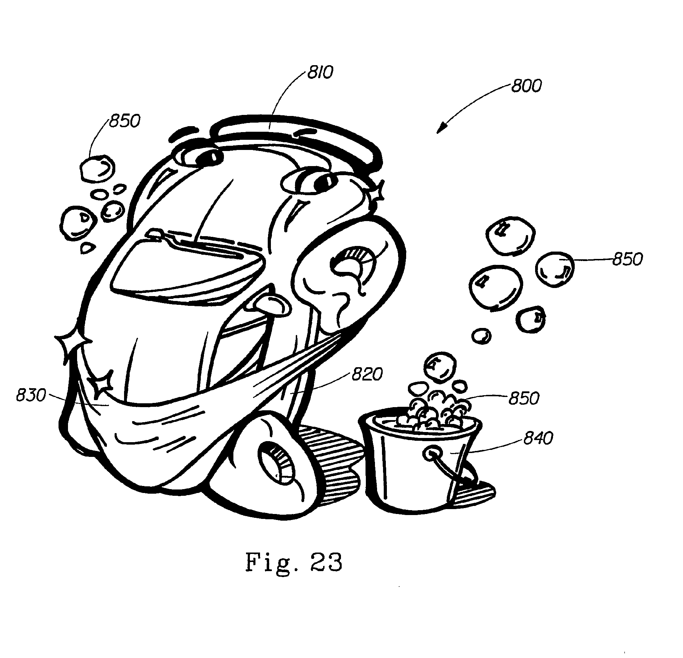

Figure 23 illustrates another child graphic.

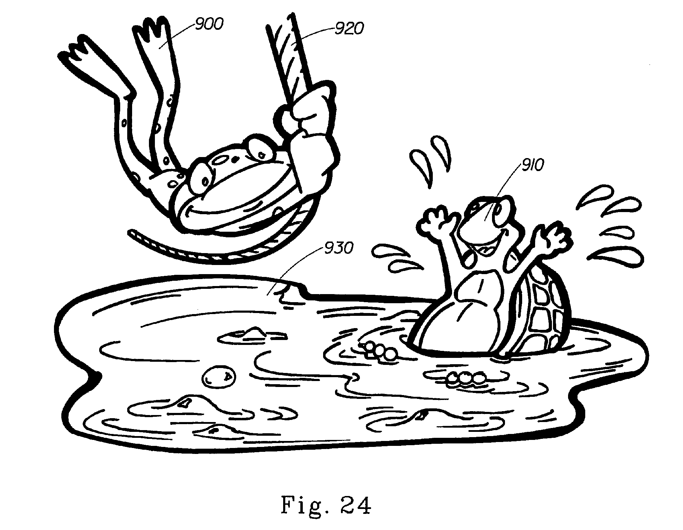

Figure 24 illustrates another child graphic.

### DETAILED DESCRIPTION

The instant implements, and methods of the present invention are suitable for use by children in personal cleansing. Due to the ease and simple method of use very young children are able to clean themselves, to an extent independently, with the instant invention.

### DEFINITIONS

As used herein the abbreviation "gsm" means "grams per square meter".

As used herein, "disposable" is used in its ordinary sense to mean an implement that is disposed or discarded after a limited number of usage events, preferably about 2 or less, and more preferably about 1 entire usage events.

The term "releasably carrying" means that a composition is contained in and/or on an implement, mitt, nonwoven member and/or parts thereof and is readily releasable from the implement, mitt, nonwoven member and/or parts thereof by application of water and/or application of some force thereto, for example, wringing the disposable child sized implement, wiping a child, or immersing part or all of the disposable child sized implement in water.

As used herein, the term "comprising" means that the various components, ingredients, or steps, can be conjointly employed in practicing the present invention. Accordingly, the term "comprising" is open-ended and encompasses the more restrictive terms "consisting essentially of" and "consisting of." Other terms may be defined as they are discussed in greater detail herein.

As used herein, the term "graphic" means any design, shape, pattern, or the like that is or becomes visible on an implement, and specifically includes text messages, that include one or more alphanumeric symbol, pictorial images that consist of one or more pictures, and combination thereof.

As used herein, the term "child graphic" means any graphic which appeals to a child such that the child will want to possess and/or interact with the implement in some fashion, preferably the typical use to which the implement is put. The child graphic can be aesthetically pleasing, objectively and/or subjectively desirable to any child. The child graphic can in addition be aesthetically pleasing and/or objectively desirable to a child's caregiver. Typically, any child graphic can be supportive and/or encouraging of a child. This support and/or encouragement can be of any suitable subject matter, such as but not limited to, providing advice to the child on any of a range of diverse subjects such as: education e.g. numbers, letters, words, shapes and the like, child appropriate facts and factoids, and combinations thereof; sports and games; jokes, rhymes, limericks humorous stories and the like; social and religious issues, such as but not limited to, sharing and caring, bullying, civics, and the like; safety, such as but not limited to, stranger danger, road safety, hygiene, (i.e. hand washing, bottom wiping and the like); and combinations thereof. One such suitable subject matter of the support and/or encouragement can be with respect to the child's desire to possess and/or interact with the implement in some fashion.

A child graphic can comprise a character or characters. This character may be shown using the implement in an appropriate fashion. The child graphic may additionally include one or more secondary images of the character or characters or parts thereof, performing one or more steps associated with using the implement. Illustrative examples of such step(s) include, but are not limited to: preparation step(s) associated with using the implement, such as but not limited to accessing the implement and the like; lathering the implement and the like; using the implement; and/or disposing of the spent implement and/or optional container and the like.

Without wishing to be limited to the specific embodiments listed, suitable examples of child graphics may include: a character graphic operating a vehicle, and another child graphic comprising stars, balls, or the like; a character graphic jumping rope, and another child graphic comprising flowers; a character graphic feeding or nurturing and animal, and another child graphic comprising letters of the alphabet; a character graphic holding or using a racquet, bat, glove, other sporting equipment, or illustrated on a sporting field, or the like, and another child graphic comprising objects that are not associated with sports, sporting equipment or the like; a character graphic holding a butterfly net or the like and another child graphic comprising objects that are not associated with butterflies or the like; a character graphic holding a fishing pole, sitting in a boat or the like and another child graphic comprising objects that are not associated with fish, inflatable water toys or the like; a character graphic holding flowers, plants, gardening tools or the like and another child graphic comprising objects that are not associated with flowers, plants or gardening; a character graphic comprising a pet or other animal or an anthropomorphous image feeding, training or nurturing an animal and another child graphic comprising objects that are not associated with pets, animals, animal food, pet toys, or the like; a character graphic playing in a specific environment such as a doll house, barn yard or the like and optionally another child graphic; a character graphic holding or using a telescope or the like and another child graphic comprising objects such as stars, planets or the like; a character graphic comprising a racecar and another child graphic associated with racing; a character graphic comprising a submarine and another child graphic comprising objects associated with fish, bubbles, shells or the like; or other suitable graphics.

The child graphic may vary depending upon the age and/or developmental stage of the child. Typically, this would mean when a graphic is intended for a younger child, typically of approximate age 3 or 4, the graphics will be simpler in nature and comprise bright colors, and typically be easily identifiable and relatable to by a child of that age. The selection of available colors as well as the possible complexity of the child graphics may be increased as the age of the intended child increases. Typically, the older the intended child the more colors, especially subtle colors shades etc, and complex images are available for use on the implement.

The child graphic may vary depending upon the gender of the intended child; for example, the child graphic may comprise colors and images which are appealing to girls, such as pinks and images of dolls, rabbits, doll houses and the like or the child graphic may comprise colors and images which are appealing to boys, such as blues and rockets, construction machines, trains and the like. Alternatively, the child graphic may comprise colors and images which are gender neutral and are appealing equally to girls and boys such as purples and greens and cartoon characters, or the child graphic may comprise colors and images which comprise parts which are appealing to boys, parts which are appealing to girls and is overall appealing to both boys and girls.

The term "unrelated in subject matter" is used herein to mean that one graphic is not the same as or is not associated with the subject matter of another graphic. The subject matter relationship or lack thereof can be between two or more text messages, between two or more pictorial images, or between a combination of one or more text messages and one or more pictorial images. The term "text message" means a graphic consisting of one or more alphanumeric symbols, and the term "pictorial image" means a graphic consisting of one or more pictures. The terms "text image" and "pictorial image" are mutually exclusive as used herein.

By way of illustration and without wishing to be limited to the enumerated examples, two pictorial images are considered unrelated in subject matter where the images: illustrate items that are neither identical nor different sizes, shapes, or colors of a common object; illustrate two objects that are not commonly associated with one another, such as an animal and a building block, a jump rope and a flower, a car and a star, a letter of the alphabet and a water toy, a fish and an apple, illustrate items used in unrelated activities, such as items used in sporting activities and items used in gardening activities, or other unrelated activities; or the like. Similarly, two text messages are considered unrelated in subject matter where the messages: are neither identical nor jointly form a sentence, thought, or action; refer to two items that are not commonly associated with one another, such as "ball" and "flower," "fish" and "pencil," "car" and "ghost," or other such unrelated words; or the like. Likewise, a text message and a pictorial image are considered to be unrelated in subject matter where the text does not name, define, describe or otherwise relate to the image.

As used herein, the phrase "related in subject matter" refers to the situation where the subject matter of one graphic is the same as or is associated with the subject matter of another graphic. By way of example, two pictorial images are considered related in subject matter where the images are identical; separately illustrate different sizes, shapes, colors of a common object; each illustrate one and the other of two objects that are commonly associated with one another, such as the moon and stars, a body of water and water toys, a sandbox and suitable toys, a baseball bat and ball, a barn and animals, or the like; illustrate different items used in a particular activity, such as a sporting activity, a gardening activity or the like; jointly illustrate geometrically mating or engaging elements such as a triangle and a triangularly-shaped aperture, or two halves of a zipper; each illustrate one part of a multipart picture; or the like. Similarly, two text messages are considered related in subject matter where the messages: are identical; jointly form a sentence, thought, or action such as "jump" and "up"; each refer to one and the other of two items that are commonly associated with one another, such as but not limited to "bat" and "ball; jointly present a question and answer; or the like. Likewise, a text message and a pictorial image are considered to be related in subject matter where the text names, defines or describes the image; or the like.

The term "disposed on" and variations thereof are intended to mean that one element can be integral with another element, or that one element can be a separate structure bonded to or placed with or placed near another element. For example, graphics can be formed or applied directly or indirectly to a surface of a substrate, such as but not limited to, the nonwoven sheet member, any surface of a container, or other variations or combinations thereof. In particular embodiments, graphics may be printed, sprayed, or otherwise applied directly on a layer of the nonwoven sheet member.

### Disposable Cleaning implement

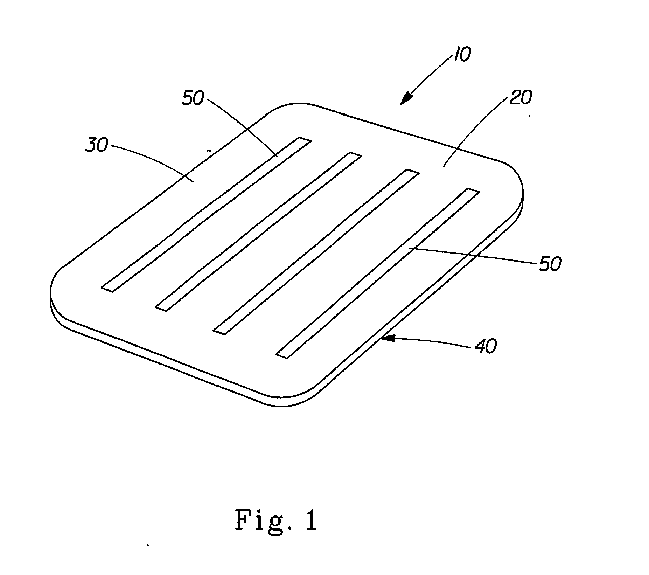

Referring to Figure 1, there is illustrated one possible embodiment of a disposable child sized cleaning implement (or disposable cleaning implement, or disposable implement, or implement) 10, in accordance with the present invention. The disposable cleaning implement 10 comprises a nonwoven sheet member, having a first surface 30, and an opposing second surface 40.

The disposable cleaning implement 10 also comprises a personal care composition 50. The disposable cleaning implement 10 is releasably carrying the personal care composition 50 on its first surface 30. In one embodiment of the present invention the personal care composition may be present on a part of the disposable cleaning implement 10, such as but not limited to, the first surface 30 in the form of stripes, spots, geometric patterns, non-geometric patterns or in a random distribution. In an alternative embodiment, the personal care composition 50 may be present on the entire first surface 30 of the disposable cleaning implement 10. In another an alternative embodiment, not shown, the personal care composition may be present in the interior of the disposable cleaning implement, and/or the first surface of the disposable cleaning implement.

It is to be understood that while in Figure 1 the first surface 30 of the disposable cleaning implement 10 is releasably carrying the personal care composition 50 in other embodiments of the present invention the opposing second surface 40 of the disposable cleaning implement 10 may be releasably carrying the personal care composition. There is no restriction as to which surface of the disposable cleaning implement is releasably carrying the personal care composition. It is even possible that all of the surfaces of the disposable cleaning implement be releasably carrying the personal care composition. Furthermore, the personal care composition may be carried on any surface, and/or interior of a disposable cleaning implement as long as the disposable cleaning implement is releasably carrying it.

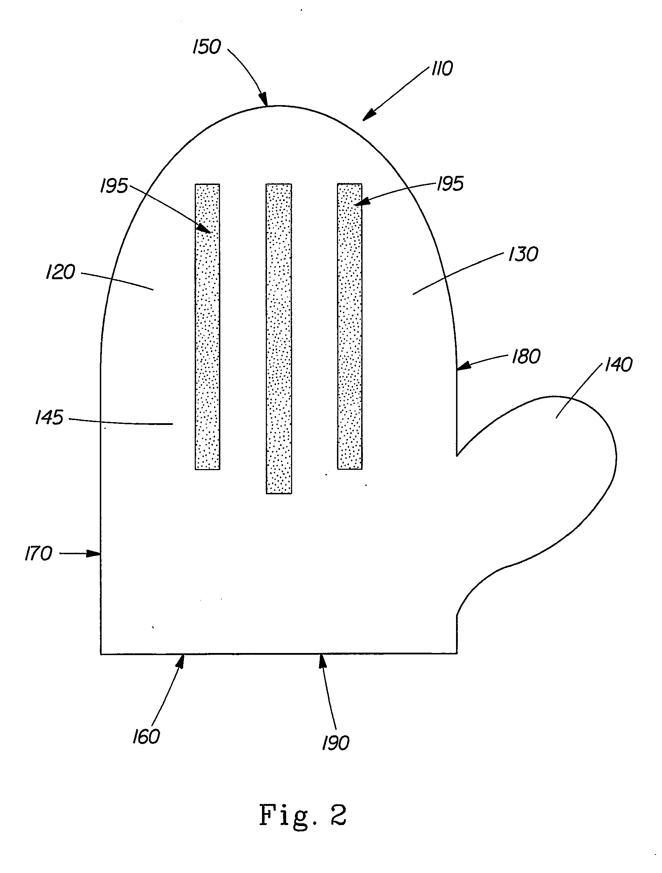

In one optional embodiment the disposable cleaning implement is a child's cleansing mitt, such as a disposable nonwoven child's cleansing mitt, and comprises first and second complementary nonwoven sheet members. Nonlimiting examples of such child's cleansing mitts may be found in Figures 2 and 4.

In Figure 2, there is illustrated one possible embodiment of a nonwoven child's cleansing mitt (or nonwoven mitt, or mitt or mitten) 110, in accordance with the present invention. The mitt 110 comprises a first nonwoven sheet member 120, which has an exterior surface 130, an interior surface, a top edge 150, a bottom edge 160, a first side edge 170 and a second side edge 180. The first nonwoven sheet member 120, together with a substantially complementary second nonwoven sheet member, which are in an overlying relationship, define an interior volume which is accessed by the user's hand (i.e. a child) via opening 190.

The interior volume of mitt 110 is divided into two parts, one part for the child's thumb 140 and one part for the remainder of the child's hand 145.

The mitt 110 also comprises a personal care composition 195. The first nonwoven sheet member 120 is releasably carrying the personal care composition 195 on its exterior surface 130. In one embodiment of the present invention the personal care composition may be present on a part of the first nonwoven sheet member 120, such as, but not limited to, the exterior surface 130 in the form of stripes, spots, geometric patterns, non-geometric patterns or in a random distribution. In an alternative embodiment, the personal care composition 195 may be present on the entire exterior surface 130 of the first nonwoven sheet member 120. In another an alternative embodiment, not shown, the personal care composition may be present in the interior of the first nonwoven sheet member, and/or the exterior surface of the nonwoven sheet member.

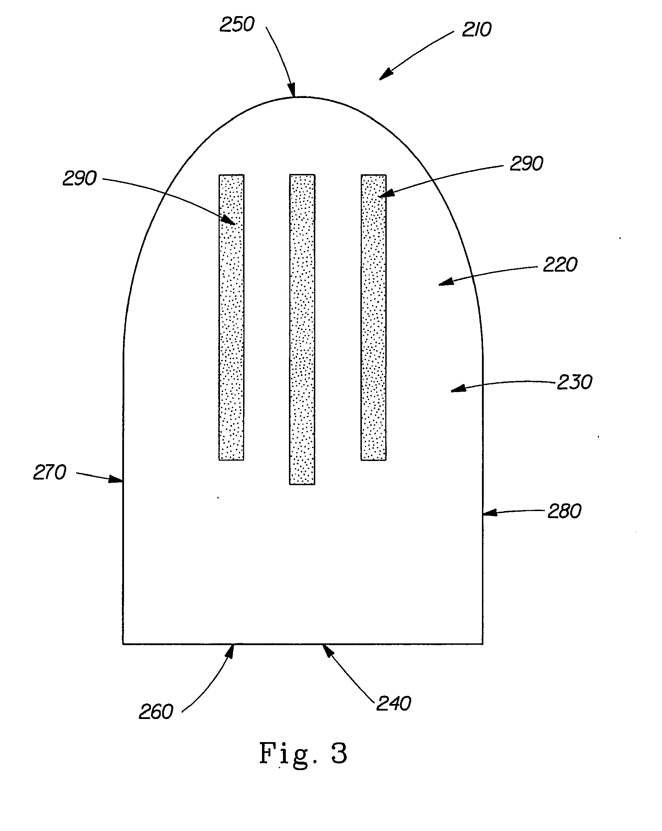

It is to be understood that while in Figure 3 the exterior surface 130 of the first nonwoven sheet member 120 is releasably carrying the personal care composition 195 in other embodiments of the present invention the second nonwoven sheet member may be releasably carrying the personal care composition. There is no restriction as to which of the first nonwoven sheet member and the second nonwoven sheet member is releasably carrying the personal care composition. It is even possible that both the first and second nonwoven sheet members be releasably carrying the personal care composition. Furthermore, the personal care composition may be carried on the exterior surface, interior, and/or interior surface of a nonwoven member as long as the nonwoven member is releasably carrying it.

Referring to Figure 3, there is illustrated another possible embodiment of a mitt 210, in accordance with the present invention. The mitt 210 comprises a first nonwoven sheet member 220, which has an exterior surface 230, an interior surface, a top edge 250, a bottom edge 260, a first side edge 270 and a second side edge 280. The first nonwoven sheet member 220, together with a complementary second nonwoven sheet member, which are in an overlying relationship, define an interior volume which is accessed by the user's hand via opening 240.

The mitt 210 also comprises a personal care composition 290. The first nonwoven sheet member 220 is releasably carrying the personal care composition 290 on its exterior surface 230. In one embodiment of the present invention the personal care composition may be present on a part of the first nonwoven sheet member 220, such as but not limited to, the exterior surface 230 in the form of stripes, spots, geometric patterns, non-geometric patterns or in a random distribution. In an alternative embodiment, the personal care composition 290 may be present on the entire exterior surface 230 of the first nonwoven sheet member 220. In another an alternative embodiment, not shown, the personal care composition may be present in the interior of the first nonwoven sheet member, and/or the exterior surface of the nonwoven sheet member.

It is to be understood that while in Figure 3 the exterior surface 230 of the first nonwoven sheet member 220 is releasably carrying the personal care composition 290 in other embodiments of the present invention the second nonwoven sheet member may be releasably carrying the personal care composition. There is no restriction as to which of the first nonwoven sheet member and the second nonwoven sheet member is releasably carrying the personal care composition. It is even possible that both the first and second nonwoven sheet members be releasably carrying the personal care composition. Furthermore, the personal care composition may be carried on the exterior surface, interior, and/or interior surface of a nonwoven member as long as the nonwoven member is releasably carrying it

In one optional embodiment of the present invention the disposable cleaning implement of the present invention may comprise an attachment means to snugly attach the disposable cleaning implement to the child's hand. The attachment means may be any suitable means for permitting a removable attachment of the disposable cleaning implement of the present invention to a child's hand. Suitable adjustment means, include but are not limited to, hook and loop fasteners such as Velcro® and the like, elastic members, buttons, fasteners, tabs, resealable tape, belts, clips, refastenable adhesives, and combinations thereof.

In one optional embodiment of the present invention when the disposable cleaning implement is a child's cleansing mitt, it may comprise an adjustment means to snugly accommodate different child hand sizes. The adjustment means may be any means suitable for permitting a variation in the size of an interiorly defined volume, to snugly accommodate different child hand sizes. Suitable adjustment mechanism, include but are not limited to, include, hook and loop fasteners such as Velcro® and the like, gussets, cinches, elastic members, elastic strands, buttons, fasteners, tabs, resealable tape, belts, clips, refastenable adhesives, and combinations thereof.

In an alternative embodiment of the present invention the material, or a portion of the material, which comprises the disposable cleaning implement is selected such that it, will adhere, cling or stick to the child's hand prior to and during use. For example, the mitt 210 of Figure 3, may optionally comprise in its interior surface material that will adhere, stick or cling to the child's hand prior to and during use. This optional adhesion may be achieved in a variety of ways, including but not limited to, adhesive, friction, electrostatic attraction, conformation or constriction of the disposable cleaning implement or a portion thereof, to the shape of the child's hand when wet, fluid between the child's hand and the material and combinations thereof. Suitable material for this optional adhesion, include but do not limited to, adhesive, polyolefin films such as films comprising polyethylene and/or polypropylene, and combinations thereof.

The material of which disposable cleaning implements are made from should be strong enough to resist tearing during normal use, yet still provide softness to the child's tender skin. Additionally, the material should be water insoluble, or at least capable of retaining its form for the duration of the child's cleansing experience.

In one embodiment of the instant invention the disposable cleaning implements comprise a mixture of natural fibers and synthetic fibers. In alternative embodiments of the present invention the disposable cleaning implements may wholly comprise natural fibers, while in other alternative embodiments still may wholly comprise synthetic fibers.

In one embodiment of the present invention when the disposable cleaning implement comprises two nonwoven sheet members each nonwoven sheet member is made of material which is different to that of the other nonwoven sheet member. In another alternative embodiment of the present invention when the disposable cleaning implement comprises two nonwoven sheet members the two nonwoven sheet members are made of the same material.

Suitable natural fibers include but are not limited to cellulosic fibers, such as wood pulp fibers, cotton, and rayon. Suitable synthetic fibers include fibers commonly used in textiles, including but not limited to, polyester and polypropylene fibers polyethylene, polyether, PET, and combinations thereof. It is also possible to use bicomponent polymers, or simply bico or sheath polymers. These bico polymers can be used as a component fiber of the nonwoven sheet member, and/or they may be present to act as a binder for the other fibers present in the nonwoven material. Suitable nonwovens with good softness include, but are not limited to, nonwoven materials comprising polypropylene, polyethylene, cellulose, rayon, polyether, PET, bicomponent polymers, and combinations thereof.

Various forming methods can be used to form the disposable cleaning implements, and/or the nonwoven sheet members. For instance, the disposable cleaning implements can be made by nonwoven dry forming techniques, such as air-laying, or alternatively by wet laying, such as on a papermaking machine, of a continuous web out of which the cleansers are made. Other nonwoven manufacturing techniques, including but not limited to, techniques such as adhesive bonding, melt blown, spunbonded, carding, needle punched, hydroentanglement and lamination methods may also be used.

The disposable cleaning implements of the present invention may be subjected to various treatments, such as but not limited to, physical treatment, such as zone activation, ring rolling SELFing and the like; chemical treatment, such as rendering part or all of the disposable cleaning implements hydrophobic, and/or hydrophilic, and the like; thermal treatment, such as softening of fibers by heating, thermal bonding and the like; and combinations thereof.

The disposable child sized implement may be of any size which is suitable for use by a child. Furthermore, the implement may be of a size to be used by a specific developmental age of child, such as 4, or 7and the like. Alternatively, the disposable child sized implement may be of a size which is suitable for use by any child. Typically, the size of the disposable child sized implement will depend upon many factors such as dexterity and hand eye coordination of the child, size of the child's hand, gender, ethnicity, and the like. Anthropomorphic data on child hand sizes may be found in Consumer Safety CHILDATA: Handbook of Child Measurements (Beverly Norris & John Wilson, June 1985). Furthermore, the dimensions and size of the disposable child sized implement will depend upon the shape, weight and composition of the nonwoven sheet member, the retaining aid used and the intended use of the implement. Typically, a substantially rectangular disposable child sized implement will have a length of from about 50 mm to about 200 mm, a width of from about 50 mm to about 150 mm and a thickness of about 0.01 mm to about 30 mm. Alternatively, the area of one side of the disposable child sized implement, such as but not limited to the side comprising the benefit composition, has an area of from about 100 mm² to about 30,000 mm².

The disposable child sized implement may also optionally comprise a usage indicator. This optional usage indicator provides a means for the child to readily identify correct usage of the implement, when all or a portion benefit composition has been released from any nonwoven member present, and/or they have used the implement for a sufficient amount of time. The usage indicator may be a separate feature or it may be part of the benefit composition, or it may be a part of a child graphic, or a child graphic when more than one child graphic is present. This type of usage indicator is described further herein. Other suitable usage indicators include, but are not limited to, pH (e.g. at a specific pH or pH range a noticeable event occurs such as color change, noise generation or cessation and the like and combinations thereof), temperature (e.g. the implement may feel warm cold for its intended use and then revert to ambient temperature, or change temperature form ambient after a period of time), time (e.g. the indicator may change size shape, color etc after a time period since it was exposed to water air, oxygen, shear or other force and the like), and the like and combinations thereof. In one optional embodiment the usage indicator provides a visual signal during use of the implement at least a portion of the benefit composition has been released, such as but not limited to, from a nonwoven member.

When present, the type of optional usage indicator will depend upon many factors, such as but not limited to, size and type of material present in implement, benefit composition, intended use of the implement, age of child using the implement the child graphic used and the like. In any event the selection of the usage indicator, when present, should not typically not be in isolation from the other elements, such as but not limited to, any character graphic, for example a usage indicator which changes to red, is probably not suitable for younger children because of the possible distress it may possibly cause to a care giver, who thinks the child is possibly hurt, and/or to the child who may think the character is possibly hurt, or a use indictor which changes to green may possibly appear to be gross and slimy to care givers and/or girls (but which may conversely possibly be fascinating and very appealing to boys). In any event selection of usage indicator will depend upon many factors and should not typically be made in isolation of the other components of the implement.

It is also within the scope of the present invention that the disposable cleaning implements, or components thereof, such as but not limited to nonwoven sheet members when present, may comprise laminates of two or more substrates or webs. Commercially available laminates, or purpose built ones would be within the scope of the present invention. Additionally, the disposable cleaning implements, and nonwoven sheet members, when present, may be flat or textured. The formation of textured disposable cleaning implements, and nonwoven sheet members, when present, and laminates forms no part of this invention. The following discussion is for convenience of formulation, but is not intended to limit the type of disposable cleaning implements used herein.

In one embodiment of the present invention the surface of disposable cleaning implements is essentially flat. In another embodiment of the present invention the surface of the cleanser may optionally contain raised and/or lowered portions. These can be in the form of logos, indicia, trademarks, geometric patterns, images of the surfaces that the child's cleansing mitt is intended to clean (i.e. child's body, face, etc.,). They may be randomly arranged on the surface of the disposable cleaning implements or be in a repetitive pattern of some form. They may be on one or both of the sides or surfaces of the disposable cleaning implements. In one embodiment one of the nonwoven sheet members contains a repetitive pattern or alternating raised and lowered portions of the substrate. This variation in or on the surface of one side of the disposable cleaning implement may be included to, for example, convey to the child or a caregiver information on the disposable cleaning implements intended use, how a child is to place the child's cleansing mitt on the child's hand, which brand or type of disposable cleaning implement they are using is or even to aid in cleaning of the child.

In another embodiment of the present invention the disposable cleaning implements is biodegradable. For example the cleanser could be made from a biodegradable material, such as a polyesteramide.

In one optional embodiment the disposable cleaning implement is a child's cleansing mitt, and comprises first and second substantially complementary nonwoven sheet members. The first and second complementary nonwoven sheet members may be joined or bonded together in any suitable fashion. For example, the first and second nonwoven sheet members may be joined by ultrasonically bonding, sewing, adhesively, mechanically bonding, fusion bonding, heat or thermal bonding and combinations thereof. The first and second nonwoven sheet members are joined at their respective first, second and top edges. The bottom edges may be either totally unbonded or partially bonded. Any such partial bond will not restrict a child from wearing the mitt, and may aid in securing the mitt to the child's hand.

Additional information on materials which are suitable for use as the disposable cleaning implements, child's cleansing mitt, nonwoven sheet members and/or other components thereof can be found in the following patents: U.S. Patent 3,862,472 issued Jan 28, 1975; U.S. Patent 3,982,302 issued Sept. 28, 1976; U.S. Patent 4,004,323 issued Jan. 25, 1977; U.S. Patent 4,057,669 issued Nov. 8, 1977; U.S. Patent 4,097,965 issued July 4, 1978; U.S. Patent 4,176,427 issued Dec. 4, 1979; U.S. Patent 4,130,915 issued Dec. 26, 1978; U.S. Patent 4,135,024 issued Jan. 16, 1979; U.S. Patent 4,189,896 issued Feb. 26, 1980; U.S. Patent 4,207,367 issued June 10, 1980; U.S. Patent 4,296,161 issued Oct. 20, 1981; U.S. Patent 4,309,469 issued Jan 25, 1982; U.S. Patent 4,682,942 issued July 28, 1987; U.S. Patents 4,637,859; 5,223,096; 5,240,562; 5,556,509; and 5,580,423 and U.S. Patent Application No. US2003/0217425 Published on November 27, 2003 and filed on May 23, 2002 by Datta et al.

Additional information on suitable disposable child sized implement, nonwoven sheet members and/or retaining aids may be found in copending U.S. Patent Application Nos US 2004/0204333 filed on March 10, 2003, entitled "Disposable Nonwoven Cleansing Mitt" in the name of Dobrin et al; US 2005/0220847 filed on March 10, 2003, entitled "Disposable Nonwoven Cleansing Mitt" in the name of Benjamin et al; and US 2005/0150784 filed on March 10, 2003, entitled "Child's Cleansing System" in the name of Sanchez et al.

The manufacture of disposable cleaning implements, mitts, nonwoven sheet member and components thereof, such as, nonwoven sheet substrate per se forms no part of this invention.

### Personal Care Compositions

The personal care compositions releasably carried by the disposable cleaning implement of the present invention may comprise a variety of components such as are conventionally used in personal care compositions. These optional components should be suitable for application to a child's skin and hair; that is, when incorporated into the implement they are suitable for use in contact with human skin without undue toxicity, incompatibility, irritation, instability, allergic response, and the like, within the scope of sound medical or formulator's judgment.

In one embodiment of the present invention the personal care compositions are in the form of a paste, or a dry solid. While personal care compositions comprising more than about 50% by weight of the composition of a liquid carrier, such as water, are within the scope of the present invention, it is preferred that any disposable cleaning implement be mostly dry, more preferably dry to the touch, prior to contact with the washing environment, that is, until the child first immerses the disposable cleaning implement or otherwise contacts it with water. Typically, this translates into levels of liquid carrier, such as water, of less than or equal to about 10%, more preferably less than or equal to about 7% by weight of personal care composition.

In one alternative embodiment of the present invention the amount of personal care composition present in the disposable cleaning implement is preferably present in amounts from about 1 gsm to about 200 gsm, more preferably from about 10 gsm to about 175 gsm, even more preferably still from about 20 gsm to about 150 gsm, (Grams of personal care composition per square meter of nonwoven sheet member) Alternatively, each disposable cleaning implement may contain from 1g to 20g, more preferably from 1g to 15g of personal care composition per disposable cleaning implement.

### Biological extract

The personal care compositions comprise a biological extract selected from the group consisting of:
(i) a sterol;
(ii) an animal extract;

While not wanting to be limited by theory, it is believed that the biological extract enhances skin barrier repair and moisture balance of the skin and disposable implement releasably carrying such an extract would leave the skin feeling soft and supple be able to at least mitigate, if not reverse, any damage done to a child's skin, such as reducing skin hydration and the like.

The amount biological extract present in the personal care composition will depend upon many factors, such as but not limited to, other components of the personal care composition, the implement, and the like, Preferably, the biological extract is present in the personal care composition in the range of from 0.001% to 50%, more preferably from 0.005 to 25%, even more preferably from 0.01 to 10% by weight of the personal care composition.

### (i) Sterol

In one optional embodiment the biological extract may comprise a sterol. The sterol or sterol derivatives of may be any suitable sterol, such as but not limited to, one or more of the group consisting of: β-sterols having a tail on the 17 position and having no polar groups, for example cholesterol, sitosterol, stigmasterol, and ergosterol, as well as, C₁₀ -C₃₀ cholesterol/lanosterol esters, cholecalciferol, cholesteryl hydroxystearate, cholesteryl isostearate, cholesteryl stearate, 7-dehydrocholesterol, dihydrocholesterol, dihydrocholesteryl octyidecanoate, dihydrolanosterol, dihydrolanosteryl octyldecanoate, ergocalciferol, tall oil sterol, soy sterol acetate, lanasterol, soy sterol, avocado sterols, cholesterol esters, sterol esters, avocadin, lanolin, and the like.

While not wanting to be limited by theory, sterols are believed to facilitate repair to damaged skin. The amount of sterol present in the personal care composition will depend upon many factors, such as but not limited to, other components of the personal care composition, the implement, and the like. In one optional embodiment, the sterol is present in the personal care composition in the range of from 0.001% to 50%, more preferably from 0.005 to 25%, even more preferably from 0.01 to 10% by weight of the personal care composition.

### (ii) Animal extract

In one optional embodiment the biological extract may comprise an animal extract. The term, "animal extract" means an adjunct which comprises the product of an extraction mechanical separation or combinations thereof, such as but not limited to steam distillation, hydrolysis, grinding, mastication, aqueous extraction, rendering, and the like and combinations thereof, of animal material, i.e. animals or parts thereof. The extract may comprise a single compound, or it may be a mixture of compounds. The animal extract maybe treated or further processed, such as but not limited to de-colorization, bleaching, deodorization, hardening and the like, prior to incorporation into the personal care composition. The term "animal extract" does not include essential oils, or other so-called aromatherapy adjuncts. The term "animal" means any vertebrate or invertebrate, which is incapable of performing photosynthesis, such as but not limited to mammals (e.g. llamas and the like), reptiles (e.g. crocodiles and the like), amphibians (e.g. frogs and the like), birds (e.g. albatrosses and the like) fish (e.g. sharks and the like), insects (e.g. stick insects and the like) and the like.

Some illustrative animal extracts include but are not limited to: fats and oils such as, but not limited to, Cod Liver Oil, Egg Oil, Human Placental Lipids, Hydrogenated Fish Oil, Hydrogenated Lard, Hydrogenated Orange Roughy Oil, Hydrogenated Shark Liver Oil, Lard, Orange Roughy Oil, Phospholipids, Placental Lipids, Shark Liver Oil, Tallow, and the like, and mixtures thereof; fatty acids, such as but not limited to, Hydrogenated Tallow Acid, Hydroxystearic Acid, Isostearic Acid, and the like, as well as mixtures thereof; and fatty alcohols, such as but not limited to, Hydrogenated Tallow Alcohol, Stearyl Alcohol, Tallow Alcohol, and the like, as well as mixtures thereof.

In another optional embodiment the animal extract comprises a protein. In one optional embodiment the animal extract comprises silk protein and/or derivatives thereof, hydrolyzed animal collagen protein, milk protein and/or derivatives thereof, and combinations thereof.

One such protein is sericin. Sericin is one of two proteins that are part of the twin fibroin silk thread spun by Bombyx mori, a domestic insect. Sericin acts as a protective envelope around the fibroin thread as it is spun, which is like spinning of fibers with soluble sizing agents to help form good quality fibers. The sericin can be easily separated from silk protein by hydrolysis. Post-spun sericin, with its unique properties, while not wanting to be limited by theory, is believed to have high affinity to a number of proteins. When refined to a high molecular weight substance, while not wanting to be limited by theory, is believed to be amenable to binding to the keratin of skin and hair, forming a resistant, moisturizing, and protective film on the skin/hair, imparting good barrier properties.

Sericin is a silk protein obtained by controlled hydrolysis of low molecular weight silk having a specific gravity of at least about 1. A commercially available silk protein is available from Croda, Inc., of Parsippany, N.J., and is sold under the trade name CROSILK LIQUID (silk amino acids), CROSILK 10,000 (hydrolyzed silk), CROSILK POWDER (powdered silk), and CROSILKQUAT (cocodimonium hydroxypropyl silk amino acid). Another example of a commercially available silk protein is SERICIN, available from Pentapharm, LTD, a division of Kordia, bv, of the Netherlands. Further details of such silk protein mixtures can be found in U.S. Pat. No. 4,906,460, to Kim, et al., assigned to Sorenco.

Silk protein derivatives are also suitable for use in the personal care compositions. Silk protein derivatives may be chosen from one of several potential compositions. Included among the silk derivatives are silk fibers and hydrolysate of silk fibers. The silk fibers may be used in the form of powder in preparing the emulsion or as a powder of a product obtained by washing and treating the silk fibers with an acid. Preferably, silk fibers are used as a product obtained by hydrolysis with an acid, alkali or enzyme, as disclosed in U.S. Pat. No. 4,839,168 to Abe et al.; U.S. Pat. No. 5,009,813 to Watanube et al., and U.S. Pat. No. 5,069,898 to Goldberg.

Another silk derivative that may be employed is protein obtained from degumming raw silk, as disclosed, for example, in U.S. Pat. No. 4,839,165 to Hoppe et al. The principal protein obtained from the raw silk is sericin, which has an empirical formula of C₁₅ H₂₅ O₃ N₅ and a molecular weight of 323.5.

One suitable silk derivative is a mixture of two or more individual amino acids, which naturally occur in silk. The principal silk amino acids are glycine, alanine, serine and tyrosine.

Another example of a silk derivative for use in the personal care composition is a fine powder of silk fibroin in nonfibrous or particulate form, as disclosed in U.S. Pat. No. 4,233,212 to Otoi et al.

The fine powder is typically produced by dissolving a degummed silk material in at least one solvent selected from, for example, an aqueous cupriethylene diamine solution, an aqueous ammonia solution of cupric hydroxide, an aqueous alkaline solution of cupric hydroxide and glycerol, an aqueous lithium bromide solution, an aqueous solution of the chloride, nitrate or thiocyanate of calcium, magnesium or zinc and an aqueous sodium thiocyanate solution. The resulting fibroin solution is then dialyzed. The dialyzed aqueous silk fibroin solution, having a silk fibroin concentration of from about 3 to 20% by weight, is subjected to at least one treatment for coagulating and precipitating the silk fibroin, such as, for example, by the addition of a coagulating salt, by aeration, by coagulation at the isoelectric point, by exposure to ultrasonic waves, by agitation at high shear rate and the like.

The resulting product is a silk fibroin gel, which may be incorporated directly into a personal care composition or the same may be dehydrated and dried into a powder and then dissolved in the personal care composition.

The silk material used to form the silk fibroin includes cocoons, raw silk, waste cocoons, raw silk waste, silk fabric waste and the like. The silk material is degummed or freed from sericin by a conventional procedure such as, for example, by washing in warm water containing a surfactant-active agent or an enzyme, and then dried. The degummed material is dissolved in the solvent and preheated to a temperature of from about 60 to 95° C., preferably of from about 70 to 850° C. Further details of the process of obtaining the silk fibroin are discussed in previously referenced U.S. Pat. No. 4,233,212.

The protein may also comprise hydrolyzed animal collagen protein, which may be obtained via any conventional process, such as but not limited to enzymatic hydrolysis and the like. Non-limiting examples hydrolyzed animal collagen protein include, lexeine protein and the like.

In another optional embodiment of animal extract may comprise milk protein and/or milk protein hydrolysate. While not wanting to be limited by theory it is believed that the, milk protein serves as a cationic conditioning agent for the skin. Suitable milk protein compounds for use in the personal care composition include but are not limited to, hydrolyzed milk protein, hydrolyzed milk protein derivative, or any combinations thereof. Suitable hydrolyzed milk protein derivatives include, for example, palmitoyl derivative and quaternary ammonium salt derivative. In one optional embodiment the milk protein is a hydrolyzed milk protein sold under the trade name Hydrolactin 25000 by Croda, Inc.

The amount animal extract present in the personal care composition will depend upon many factors, such as but not limited to, other components of the personal care composition, the implement, and the like. In one optional embodiment, the animal extract is present in the personal care composition in the range of from 0.001% to 50%, more preferably from about 0.005 to about 25%, even more preferably from 0.01 to 10% by weight of the personal care composition.

### Surfactants

The personal care compositions used in the present invention may optionally contain one or more surfactant. Typically the optional surfactant, when present, is selected from the group consisting of anionic surfactants, amphoteric surfactants, nonionic surfactants, zwitterionic surfactants, cationic surfactants, and mixtures thereof.

The surfactants of the personal care compositions may be lathering or non-lathering surfactants. As used herein, "lathering surfactant" means a surfactant, which when combined with water and mechanically agitated generates a foam or lather. A "nonlathering surfactant" produces no such foam or lather under similar conditions. It is preferred, however, that the surfactants be lathering since increased lather is important to consumers as an indication of cleansing effectiveness.

Nonlimiting examples of surfactants useful in the compositions of the present invention are disclosed in McCutcheon's, Detergents and Emulsifiers, North American edition (1986), published by Allured Publishing Corporation; McCutcheon's, Functional Materials, North American Edition (1992); and U. S. Patent No. 3,929,678, to Laughlin et al., issued December 30, 1975.

Some nonlimiting examples of suitable surfactants include ammonium lauroyl sarcosinate, sodium trideceth sulfate, sodium lauroyl sarcosinate, ammonium laureth sulfate, sodium laureth sulfate, ammonium lauryl sulfate, sodium lauryl sulfate, ammonium cocoyl isethionate, sodium cocoyl isethionate, sodium lauroyl isethionate, sodium cetyl sulfate, sodium monolauryl phosphate, sodium cocoglyceryl ether sulfonate, sodium C₉-C₂₂ soap, amine oxides such as lauramine oxide and cocoamine oxide, decyl polyglucose, lauryl polyglucose, sucrose cocoate, C₁₂₋₁₄ glucosamides, sucrose laurate, fatty amines, di-fatty quaternary amines, tri-fatty quaternary amines, imidazolinium quaternary amines, PEG 80 Sorbitan laurate, PEG-150 distearate, sodium laureth-13 carboxylate, disodium lauroamphodiacetate, sodium lauroamphoacetate, cetyl dimethyl betaine, cocoamidopropyl betaine, cocoamidopropyl hydroxy sultaine, and combinations thereof.

Surfactant, when present, is typically employed in compositions at levels of preferably from 0.01% to 99%, more preferably from 0.5% to 97 %, and more preferably from 1.0% to 98%, by weight of the personal care composition.

### Adjunct Ingredients

The personal care compositions used in the present invention may optionally contain one or more adjunct ingredients. Illustrative, but nonlimiting examples of suitable adjunct ingredients include: enzymes, absorbents, aesthetic components, fragrances, pigments, colorings, colorants, skin sensates, anti-acne agents (e.g., resorcinol, sulfur, salicylic acid, erythromycin, zinc, etc.), anti-caking agents, antifoaming agents, preservative, conditioners, hair conditioners, dye, antimicrobial agents (e.g., quaternium-15, paraben preservatives such as but not limited to ethyl paraben, DMDM hydantoin, iodopropyl butylcarbamate(IPBC) etc.), glycerin, binders, buffering agents, bulking agents, chelating agents (e.g., EDTA etc), solvents, cosmetic biocides, denaturants, external analgesics, film formers or materials, e.g., polymers, for aiding the film-forming properties and substantivity of the composition (e.g., copolymer of eicosene and vinyl pyrrolidone), humectants, polydimethylsiloxanes (such as but not limited to dimethicones), Cyclic polyalkylsiloxanes, opacifying agents, pH adjusters, process aids, reducing agents, sequestrants, skin-conditioning agents, moisturizers, skin soothing and/or healing agents (e.g., panthenol and derivatives (e.g., ethyl panthenol), flavonoids (e.g., bioflavonoids, flavones, isoflavones, etc), conditioners (e.g. hair conditioners), aloe vera, pantothenic acid and its derivatives, allantoin, bisabolol, and dipotassium glycyrrhizinate), hair detanglers, skin treating agents, thickeners (e.g. polymeric thickeners, gums, etc), hydrocolloids, zeolites, sugar amines also known as Amino sugars (e.g. glucosamine, N-acetyl glucosamine, mannosamine, N-acetyl mannosamine, galactosamine, N-acetyl galactosamine, their isomers (e.g., stereoisomers), and their salts (e.g., HCl salt)), phytosterols (e.g. β-sitosterol, campesterol, and the like), oxidants/radical scavengers (such as ascorbic acid (vitamin C), ascorbyl esters of fatty acids, ascorbic acid derivatives (e.g., magnesium ascorbyl phosphate), tea extracts (such as green tea extracts), plant and fruit extracts (e.g. grape skin/seed extracts, melanin, and rosemary extracts, Manjistha, Guggal, kola extract, chamomile, red clover extract, sea whip extract), caffeine, candelilla wax, alpha-bisabolol, aloe vera, allantoin, glycyrrhetic acid, glycyrrhizic acid, abrasives, astringents, etc. (e.g., menthol, menthyl lactate, etc), binders, gelling agents, thixatropic agents, bulking agents, cosmetic astringents, cosmetic biocides, denaturants, opacifying agents, pH adjusters, reducing agents, sequestrants, skin bleaching and lightening agents (e.g., hydroquinone), skin treating agents, sunscreen actives (e.g. p-aminobenzoic acid, 2-ethylhexyl-p-methoxycinnamate, 2,2'-dihydroxy-4,4'-dimethoxybenzophenone, zinc oxide, titanium dioxide etc,), and vitamins and derivatives thereof (e.g., tocopherol, tocopherol acetate, beta carotene, nicotinic acid, retinoic acid, retinol, retinoids, retinyl palmitate, niacin, pantothenic, niacinamide, nicotinyl alcohol, and the like). The personal care compositions releasably contained by the disposable implement may include carrier components such as are known in the art, for example water, alcohols, polyols, and the like. Such carriers can include one or more compatible liquid or solid filler diluents or vehicles which are suitable for application on to and/or use by a child. Alternatively these carriers may be present in the personal care composition during formulation and application to the implement when present and subsequently removed, by any conventional means, such as but not limited to heating, reducing air pressure, and the like.

Additional information on suitable personal care compositions, surfactants and other possible components thereof may be found in: CTFA Cosmetic Ingredient Handbook, Second Edition (1992); CTFA International Cosmetic Ingredient Dictionary, Fifth Edition, 1993; McCutcheon's, Detergents and Emulsifiers, North American edition (1986), published by Allured Publishing Corporation; McCutcheon's, Functional Materials, North American Edition (1992); Sagarin, et al., Cosmetics Science and Technology (1972); U.S. Patent No. 3,929,678, to Laughlin et al., issued December 30, 1975; U.S. Patent No. 5,833,998 issued to Biedermann et al., on November 10, 1998; U.S. Patent No. 5,939,082 issued to Oblong et al., on Augiust 17, 1999; U.S. Patent 3,755,560, issued August 28, 1973, to Dickert et al.; U.S. Patent 4,421,769, issued December 20, 1983, to Dixon et al.; U.S. Patent No. 4,960,764, to Figueroa, Jr. et al., issued October 2, 1990; U.S. Patent No. 6,335,312 issued on January 1, 2002 to Coffindaffer et al; U.S. Patent No. 5,607,980, issued to McAtee et al, on March 4, 1997; U.S. Patent No. 5,487,884, issued 1/30/96 to Bissett et al.; U.S. Patent No. 5,686,082, issued to N'Guyen on November 1, 1997; U.S. Patent No. 2,831,854, issued to Tucker et al., on April 22, 1958; U.S. Patent No. 4,005,196, to Jandacek, issued January 25, 1977; U.S. Patent No. 4,005,195, to Jandacek, issued January 25, 1977; U.S. Patent No. 5,306,516, to Letton et al, issued April 26, 1994; U.S. Patent No. 5,306,515, to Letton et al, issued April 26, 1994; U.S. Patent No. 5,305,514, to Letton et al, issued April 26, 1994; U.S. Patent No. 4,797,300, to Jandacek et al, issued January 10, 1989; U.S. Patent No. 3,963,699, to Rizzi et al, issued June 15, 1976; U.S. Patent No. 4,518,772, to Volpenhein, issued May 21, 1985; U.S. Patent No. 4,517,360, to Volpenhein, issued May 21, 1985; U.S. Patent No. 4,976,953, to Orr et al, issued December 11, 1990; U.S. Patent No. 5,087,445, to Haffey et al, issued February 11, 1992; U.S. Patent No. 4,509,949, to Huang et al, issued April 5, 1985; U.S. Patent No. 2,798,053, to Brown, issued July 2, 1957; U.S. Patent No. 5,100,660, to Hawe et al, issued March 31, 1992; U.S. Patent No. 4,849,484, to Heard, issued July 18, 1989; U.S. Patent No. 4,835,206, to Farrar et al, issued May 30, 1989; U.S. Patent No. 4,628,078 to Glover et al issued December 9, 1986; U.S. Patent No. 4,599,379 to Flesher et al issued July 8, 1986; U.S. Patent No. 6,200,554, issued to Yeoh et al., on March 13, 2001; U.S. Patent No. 6,248,317 issued to Snyder et al., on June 19, 2001; U.S. Patent No. 4,741,855 issued to Grote et al., on May 3, 1988 and re issued as USRE 34584 on April 12, 1994; U.S. Patent No. 6,280,751 issued to Fletcher et al., on August 28, 2001; U.S. Patent No. 6,506,394 issued to Yohiaoui et al., on January 14, 2003; U.S. Patent No. 6,440,437 issued to Krzysik et al., on August 27, 2002; U.S. Patent No. 6,630,175 issued to Shapiro et al., on October 7, 2003; EP 228,868, to Farrar et al, published July 15, 1987; WO 97/39733 A1, published on October 30, 1997 US. Patent Application No. US20030190337A1: "Methods for regulating the condition of mammalian keratinous tissue via topical application of vitamin B6 compositions", published on October 9, 2003; US. Patent Application No. US20030130636A1: "System for improving skin health of absorbent article wearers", published on July 10, 2003; and US. Patent Application No. US20020177535A1: "Cleansing compositions with milk protein and aromatherapy", published on November 28, 2002.

Mixtures of the above components may also be used.

Also, when applicable, the pharmaceutically-acceptable salts, of the components are useful herein.

Adjunct ingredients, when present, are each typically employed in compositions at levels of from 0.0001% to 99.9%, preferably from 0.001% to 99%, and more preferably from 0.01% to 97%, by weight of the personal care composition.

In preparing the disposable cleaning implement of the present invention the personal care composition need to be releasably carried by the disposable cleaning implement, such as placed on one of the nonwoven sheet members. Techniques for combining the disposable cleaning implement or nonwoven sheet members with the personal care composition are well known in the art. Examples of common methods of combining the personal care composition with the disposable cleaning implement may involve coating, immersing, dipping, printing, and/or spraying, a nonwoven sheet member with the personal care composition. The personal care composition of is added to the disposable cleaning implement at level sufficient to provide the desired benefits of the present invention. One illustrative convenient method of combining the personal care composition of the present invention with the disposable cleaning implement is for the personal care composition to be applied to a nonwoven sheet member while the nonwoven sheet member is a continuous web. The application could be in many forms, including one or more of, but not limited to coating, immersing, dipping, spraying, printing, extruding and the like. Once the personal care composition is applied the nonwoven sheet member is cut to the desired length to form the disposable cleaning implement and then packaged for sale. Alternatively, the personal care composition may be added to a nonwoven sheet member when the nonwoven sheet member is part of a formed mitt.

The personal care composition may be added to the disposable cleaning implement in any convenient fashion. For example, the personal care composition components could all be mixed together and then sprayed onto a nonwoven sheet member; each component could be deposited on a nonwoven sheet member separately; or half the components could be mixed together and

The personal care composition may be added to the disposable cleaning implement in any convenient fashion. For example, the personal care composition components could all be mixed together and then sprayed onto a nonwoven sheet member; each component could be deposited on a nonwoven sheet member separately; or half the components could be mixed together and then added to a nonwoven sheet member, with the remainder then being mixed together and then sprayed on to a nonwoven sheet member.

In one optional embodiment of the present invention the personal care composition is applied to the implement, in the form of a paste prior to the assembly of the disposable cleaning implement. This optional embodiment is more preferably a "hot melt" composition. Hot melt composition have high viscosity at or around room temperature, and then melt (become substantially liquid) at higher temperatures. Such systems are advantageous during processing of a disposable, substantially dry (or dry to the touch) child's cleansing mitt since the composition can be applied (e.g., coated, sprayed, extruded) to the implement at a low viscosity (e.g., a liquid) at higher than room temperature, and then as the composition cools down, it becomes a high viscosity paste or solid.

Once the personal care composition is applied to the disposable cleaning implement or components thereof, such as but not limited nonwoven sheet members, it may be further treated in any conventional manner, such as but not limited to, heating to remove excess water from the personal care composition.

### Child Graphic

The child graphic may be any suitable visual image or images. The child graphic may include pictorial symbols and/or images, such as but not limited to, photographs, such as but not limited to: a photograph of a child using the disposable child sized implement; drawings, such as a drawing of a child or an anthropomorphic image of an animal or object (See Figures 4-24) using the disposable child sized implement; cartoons, such as but not limited to, well known cartoon characters, well known brand logos or the like, or characters specifically created to be associated with the implement of commerce; symbols, such as but not limited to arrows, indications or motion or movement, and the like; and combinations thereof.

The child graphic may be arranged in any suitable fashion and may be in the form of one or more pictorial images. The arrangement may include the child graphic in, for example, a single image or picture, such as in a single image or a single cartoon. Figure 4 illustrates a single image 400 which includes a child graphic. The child graphics present in image 400 include an anthropomorphic animal 420, in this case a frog, who is in a body of water 410, holding a disposable child sized implement 430 in his hand. It is preferred that the disposable child sized implement illustrated in the child graphic or child graphics be similar in appearance, at least to a child, to any disposable child sized implement in association with the child graphic or child graphics. The image further illustrates that frog 420, is cleaning himself thereby generating suds and bubbles 450 using the disposable child sized implement 430 by contacting parts of his body 440 with the disposable child sized implement 430.

In an optional embodiment of the present invention, the child graphic is a sequential series of panels, wherein each of the panels contains, for example, a different cartoon, symbol, drawing, photograph and combinations thereof. Alternatively, each panel may contain one or more child graphic. These panels may be arranged in any suitable fashion, such as but not limited to, vertically, horizontally, diagonally, circular, and the like and combinations thereof. Examples of this optional embodiment can be found in Figures 5, 7, and 8.

In Figure 5 panels 500 are a child graphic comprising a sequential series of panels communicating to a child incapable of reading how to use a disposable child sized implement which is similar to the disposable child sized implement illustrated in Figure 2.. Panel 510 communicates where the child places its hand and the orientation of the disposable child sized implement relative to the child. Panel 520 communicates that the child needs to contact the disposable child sized implement with water, such as by immersion in a body of water such as a bath. Panel 520 also communicates to the child that the benefit composition is only present on one side of the disposable child sized implement. Additionally, panel 520 reinforces the prior communication in panel 510 on the correct orientation of the disposable child sized implement relative to the child. Panel 530, that is the frog character, additionally communicates to the child that in order to clean themselves they need to contact, such as by rubbing, scrubbing and the like, their body with the side of the disposable child sized implement which will generate lather. Additionally, panel 530 further reinforces the prior communication in panels 510 and 520 on the correct orientation of the disposable child sized implement relative to the child. Panel 540 communicates the need for the child to properly dispose of the child's cleansing mitt after they have finished bathing.

Figure 6 shows a disposable child sized implement 550 containing a repeating pattern two of the child graphics of Figure 5, namely a repeating pattern of panels 520 and 530. Disposable child sized implement 550 may be used in combination with a container which has, for example, printed thereon the entire set, or only panels 510 and 540, of Figure 5. These panels on the disposable child sized implement 550 would provide additional reinforcement to the child as to the correct use of the disposable child sized implement 550.

In Figure 7 panels 600 are a sequential series of panels each comprising a different child graphic that are communicating, especially communicating to a child incapable of reading, how to use a disposable child sized implement which is similar to the disposable child sized implement illustrated in Figure 2. Panel 610 communicates where the child places its hand to wear the disposable child sized implement and the correct orientation of the disposable child sized implement relative to the child. Panel 620 communicates not only that the child needs to contact the disposable child sized implement with water, such as by immersion in a body of water such as a bath, but that the benefit composition will generate lather when combined with water. Panel 620 also communicates to the child that the benefit composition is only present on one side of the disposable child sized implement. Additionally, panel 620 reinforces the prior communication in panel 610 on the correct orientation of the disposable child sized implement relative to the child. Panel 630 reinforces the information communicated in previous panels 610 and 620 by again communicating that the benefit composition will generate lather and is only present on one side of the disposable child sized implement. Panel 630 additionally communicates to the child that in order to clean themselves they need to contact, such as by rubbing, scrubbing and the like, their body with the side of the disposable child sized implement which will generate lather. The frog character in panel 630 communicates to the child that the disposable child sized implement is suitable for use while they are in a bath or similar body of water. Panel 640 communicates the need for the child to properly dispose of the disposable child sized implement after they have finished bathing.

In Figure 8 panels 700 are a sequential series of panels each comprising a different child graphic communicating to a child incapable of reading how to use a disposable cleaning implement. Panel 710 visually communicates that the child needs to contact the disposable cleaning implement with water, such as, by immersion in a body of water such as a bath. Panel 710 also visually communicates through arrows how a child immerses the disposable cleaning implement. Panel 720 visually communicates that the personal care composition will generate lather. Furthermore, panel 720 visually communicates through the use of a hand and motion lines, that the child needs to squeeze or exert some compressive force on the disposable cleaning implement after contact it with water to generate foam. The frog cartoon character in panel 730 communicates to the child that in order to clean themselves they need to contact, such as by rubbing, scrubbing and the like, their body with the disposable cleaning implement. Panel 730 further communicates to the child that the disposable cleaning implement is suitable for use on the child's entire body. Panel 740 communicates the need for the child to properly dispose of the disposable cleaning implement after they have finished bathing.

Figure 23 illustrates an image 800 which includes a child graphic 810. The child graphic 810 present in image 800 includes a character graphic, specifically an anthropomorphic object 820, in this case an automobile, who is standing and drying itself with a towel 830. The image 800 further includes a bucket 840 which is full of a liquid which is generating bubbles 850.

Figures 9 to 24 illustrate additional exemplary child graphics, depicting a character, in these figures frogs, monkeys, turtle or automobile in a range of various activities that a child may typically be engaged in or would readily be able to imaging themselves in that action or activity, either in place of the character or doing the activity along with the character.

The child graphic or child graphics may also include a story line in which a character, such as the as the frogs, monkeys, turtle and automobile of Figures 9 to 24, is illustrated performing an activity which will preferably lead to the character needing to perform an activity which may involve the use of the disposable child sized implement. Illustrative, but non limiting examples of such activities include, running, riding (for example riding a tricycle as illustrated in figure 9 or a bulldozer as illustrated in figure 10), playing in the mud, playing with a ball(figure 12), playing hide and seek, or other similar activities which a child does and can relate to. In this way, the child graphic or child graphics may permit the caregiver to interact with the child regarding the story line created by the child graphic or child graphics and may provide an opportunity for the caregiver to teach the child important life lessons, such as bathing and cleaning, due to the interactive nature of the child graphic.

In one alternative embodiment of the present invention when the disposable implement comprises two or more child graphics, and/or the disposable implement is present in a container which has one or more child graphics, these different child graphics may be have a common storyline.

In one alternative embodiment of the present invention when the disposable implement comprises two or more child graphics, and/or the disposable implement is present in a container which has one or more child graphics, these different child graphics may be have a related in subject matter.

Figure 24 illustrates a pair of child graphics, in this case two character graphics, a frog 900 and a turtle 910 which have a common story line and are related in subject matter. The frog 900 is swinging on a rope 920 over a pool or pond 930 in which the turtle 910 is frolicking and splashing.

In one alternative embodiment of the present invention when the disposable implement comprises two or more child graphics, and/or the disposable implement is present in a container which has one or more child graphics, these different child graphics may be unrelated in subject matter.

The child graphic may optionally include a character graphic that can increase the child's interest in using the disposable child sized implement and can increase the opportunities for the caregiver to interact positively with the child. The term "character graphic" is used herein to refer to a child graphic containing an anthropomorphous image, and in particular an image having or suggesting human form or appearance which ascribes human motivations, characteristics or behavior to inanimate objects, animals, natural phenomena, toys, cartoon characters, or the like. Ideally the character graphic would be suitable for children's swimwear, toys, clothing, diapers or the like and could be utilized to motivate children to use the disposable child sized implement. To that end, the character graphics can be associated with popular characters in the media, advertising or well known in a particular culture. Ideally they are characters that the child or caregiver care about and want to identify with. Ideally the child can imagine himself or herself taking the place of the character or emulate the character's behavior/attitude.

The role of the character graphic in the child graphic can be to encourage a child and to motivate them to behaviors, such as but not limited to, cleaning themselves, cleaning their room, and the like. The character graphic may provide a source of entertainment and reassurance for the child and a buddy, or friend, who reduces stress and can be related to in a non-competitive fashion during the training period. The character may also provide positive reinforcement and encouragement to the child while the child is learning new skills and behaviors in a non-competitive or threatening manner.

Suitable character graphics can include animals, people, inanimate objects, natural phenomena, cartoon characters or the like, that can or can not be provided with human features such as arms, legs, facial features or the like. It may be desirable for the character graphic to be familiar to the child, such as an identifiable cartoon character. The character graphics should at least be a type that the child can relate to, examples of which could include animals, toys, licensed characters, or the like. Character graphics can be made more personable and friendly to the child by including human-like features, human-like expressions, apparel, abilities, or the like. In one optional embodiment it is desirable for a character to have a distinguishing feature or features, which in a pictograph can help in training, such as a frog's webbed hand. By way of illustration, an animal character graphic can be shown smiling, wearing clothing, playing sports, fishing, driving, playing with toys, or the like. In particular embodiments, the character graphic can desirably be created to project an appearance that could be described as friendly, positive, non-intimidating, silly, independent, inspirational, active, expressive, dauntless and/or persevering. For example, the frog 420 of Figure 4 is one example of such a character graphic and is intended to inspire the child to learn how to bathe and clean themselves. The frog's expression clearly shows that while he is concentrating on cleaning himself and becoming independent, he is still smiling and having fun. Additionally, it is preferred that the characters expressions are exaggerated so as to not be too subtle for a child to understand.

Furthermore the combination of story line and character graphics are believed to make children more interested in the use of the disposable child sized implement, such as but not limited to an implement for use in a bathing or cleaning process, and therefore lead to enhanced results. While not wishing to be limited by theory it is believed that the child graphic and the other elements of the disposable child sized implement work together to provide to a child, and especially those who are incapable of reading, the appropriate tools, directions in the use of those tools and positive reinforcement which enables the child to learn how to, for example clean themselves, wash their hair, clean their room, and the like.

The character graphic, or parts thereof may retain essentially the same appearance and/or shape while the child is using the disposable implement. Alternatively, the character graphic, or parts thereof may, change appearance, shape, appear and/or disappear while the child is using the disposable implement. That is a use indicator may be optionally a part of a child graphic, or a child graphic when more than one child graphic is present. This change may occur in any suitable manner or fashion, such as but not limited to exposure to a specific environment (e.g. water, air, other suitable chemicals, a pH or pH range), time, abrasion or similar physical force or contact, and the like and combinations thereof. One example of this may be a character who is gesturing hello, welcome or the like, is changed after the child has immersed the implement in water and uses the implement, to a gesture of goodbye.

In one optional embodiment the child graphic may optionally include a character graphic which is associated with a line of children's consumer products, such as but not limited to personal cleansing products and the like. The character may be one of a family, group, team, or the like, each member of which is designed to be associated with, for example, a consumer product, a cleaning event such as washing hair, an age group, stage of infant development and the like. Alternatively, all of the characters of a family, group, team, or the like, may be designed to be associated with the entire range of consumer products.

The association by the child of the character with the consumer product, cleaning event etc., encourages and provides a way for the child to visualize through their imagination the character using the disposable child sized implement in the way intended. Furthermore, since this teaching is through the use of the child's imagination, there are none of the negative connotations associated with conventional parental instruction on how to use a consumer product, such as the disposable child sized implement. Instead of the child being subjected to parental nagging to do something the child really doesn't want to do, the child will actively use the disposable child sized implement as part of active learning play to interact with their new buddy, or friend, and imitate behavior. The interaction between the child and the character is only limited by the bounds of the child's imagination. The role of the caregiver or parent in then becomes one of actively encouraging imaginative play by the child with the character to use the disposable child sized implement correctly, instead of a being perceived by the child as a parent who stops play. Play is actively encouraged and new skills become part of play; "uninterrupted play". Since the use of the disposable child sized implement is essentially play, the child is eager to use the disposable child sized implement and learn the skill.

A family or group of character graphics can be used to progress a child through a system of consumer products, especially systems including the disposable child sized implement and the like. In this embodiment each character of the family or group, would be tailored to appeal to different groups of children. These groups may be based on age, development stages, regions, etc. Alternatively, a single character may be tailored for one particular group consumer products of line of consumer products which are different for children at different ages, development stages, etc. In this case the character may, for example be, of a different age depending on the consumer product and which group of children the product is intended to be used by.

Child graphics, such as but not limited to character graphics act to enable and encourage the desired behavior, such as the correct use of the disposable child sized implement, by providing stimuli. For example, in the case of a child graphic containing a character the stimuli may be entertainment and a friend.

### Container

In one optional embodiment of the present invention the disposable child sized implement may be present in a container. The container may be any suitable container which is capable of removably holding at least one disposable child sized implement. The container may be rigid or it may be semi-rigid. Typically, any container will have a portion for storage of the disposable child sized implement. The size of the storage portion will depend upon the many factors, such as but not limited to, the size of the disposable child sized implement(s), the number of disposable child sized implements initially present in the container, ease of use by a child, etc. This storage portion may be accessed in any suitable fashion through an opening, or orifice of a size which is suitable for the size of the disposable child sized implement.

Containers useful include but are not limited, PET tubs, flow wrap pouches, precut sachets for individually packed disposable child sized implement's, and other packaging known in the art as suitable for nonwoven implements releasably carrying a composition, such as but not limited to reach in or so called pop-up containers.

Furthermore, when present the container may be in the shape of a character, such as, but not limited to a character present in the child graphics present one or more of the disposable child sized implement.

Additional information on containers, as well as additional option components for containers, including but not limited to: container bodies; lids; containers features, such as but not limited to, attachments of lids, hinges, zippers, securing means; and the like, can be found in U.S. Patent Nos. Des 451,279 issued on December 4, 2001, to Chin; Des 437,686 issued on February 20, 2001, to Balzar; Des 443,508 issued on June 12, 2001, to Braaten; Des 443,451 issued on June 12, 2001, to Buck; Des 421,901 issued on March 28, 2000, to Hill; Des 421,902 issued on March 28, 2000, to Hill; Des 416,794 issued on November 23, 1999, to Cormack; Des 414,637 issued on October 5, 1999, to Amundson; Des 445,329 issued on July 24, 2001, to Zethoff; 3,982,659 issued on September 26, 1976, to Ross; 3,967,756 issued on July 6, 1976, to Barish; 3,986,479, issued on October 19, 1976, to Boedecker; 3,994,417 issued on November 30, 1976, to Boedecker; 6,269,970 issued on August 7, 2001, to Huang; 5,785,179 issued on July 28, 1998, to Buczwinski; 5,366,104 issued on November 22, 1994, to Armstrong; 5,322,178 issued on June 21, 1994, to Foos; 5,050,737 issued on September 24, 1991, to Josyln; 4,971,220 issued on November 20, 1990, to Kaufman; 6,296,144 issued on October 2, 2001, to Tanaka; 6,315,114 issued on November 13, 2001, to Keck; 4,840,270 issued on June 20, 1989, to Caputo; 4,471,881 issued on September 18, 1984, to Foster; 5,647,506 issued on July 15, 1997, to Julius; 6,401,968 issued on June 11, 2002, to Huang; 6,269,969 issued on August 7, 2001, to Huang; 6,412,634 issued on July 2, 2002, to Telesca; 5,791,465 issued on August 11, 1998, to Niki; 6,092,690 issued on July 25, 2000, to Bitowft; and 6,092,690 issued on July 25, 2000, to Bitowft; U.S. Patent Application Publication No. 2002/0064323 published on May 30, 2002, inventor Chin; and WO 00/27268 published on May 18, 2000, and assigned to The Procter & Gamble Co.; WO 02/14172 published on February 21, 2002, and assigned to The Procter & Gamble Co.; and WO 99/55213 published on November 4, 1999, and assigned to The Procter & Gamble Co.

While not wishing to be limited by theory it is believed that the three elements of the implement of commerce, work together to provide to a child who is incapable of reading, the appropriate tools, directions in the use of those tools and positive reinforcement which enables the child to learn how to clean themselves.

### EXAMPLES

Example 1 - A disposable child sized cleaning implement comprising:
(a) a cleaning implement which is a 105mm x 140mm rectangle comprising 60 gsm polyester nonwoven high loft batting material, Proef 1297 available from Libeltex of Meulebeke Belgium. The high loft batting material is releasably carrying the personal care composition, which comprises 50 gsm of a mixture of 1% by weight of soy sterol with the remainder being BC20, the latter of which is available from Rhodia of France; and
(b) a child graphic disposed on the implement as illustrated in Figure 5.

Example 2 - An implement according to Example 1, except that the child graphic is that illustrated in Figure 9.
Example 3 - An implement according to Example 1, except that the implement is a two-layer laminate. The first layer of the laminate is a 40gsm PET spunlace. While the second layer of the laminate is a 60 gsm polyester nonwoven high loft batting material, Proef 1297 available from Libeltex of Meulebeke Belgium. The high loft batting material is releasably carrying the personal care composition, which comprises 25 gsm of a mixture of 0.5% by weight of lanolin with the remainder being BC20, the latter of which is available from Rhodia of France.
Example 4 An implement according to Example 1, except that the personal care composition comprises 42 gsm of a composition as follows

| **Component** | **%wt.** |
|---|---|
| Sodium Laureth-3 Sulfate | 63.0 |
| Cocamidopropyl Betaine | 23.0 |
| PEG-200 Glyceryl Tallowate | 10.0 |
| Polyquatemium-10 | 1.0 |
| Preservative System | 0.5 |
| Whitener | 0.5 |
| Lanolin | 0.2 |
| Ergosterol | 0.2 |
| Perfume | 0.5 |
| Water | (Quantitiy sufficient to 100%) |

Example 5 - A disposable implement comprising:
(a) a mitt and comprising a first member which is a 60 gsm polyester nonwoven high loft bating material, Proef 1297 available from Libeltex of Meulebeke Belgium, and a second member which is a three member laminate. The three member laminate contains a 90 gsm two layer stretch laminate, namely 259-50-3 available from Tredegar, of Richmond, Virginia U.S.A., and a 30 gsm nonwoven, 008YLCO09U, available from BBA of Nashville Tennessee, U.S.A. The high loft batting material is releasably carrying the personal care composition, comprises 85 gsm of a mixture of 0.1% by weight of Hydrolactin 2500® (a hydrolyzed milk protein available from Croda Inc) with the remainder being BC20, the latter of which is available from Rhodia of France; and
(b) a child graphic as illustrated in Figure 6.

Example 6 - An implement according to Example 5, except that the child graphic is illustrated in Figure 8.
Example 7 - An implement according to Example 5, except that the mitt is the mitt of co filed application US 2005/0220847 filed on March 10, 2003, entitled "Disposable Nonwoven Cleansing Mitt" in the name of Benjamin et al., (P&G Docket Number 9180PQ).

## Claims

1. A disposable child sized cleaning implement comprising:
(a) a personal care composition, wherein said disposable child sized cleaning implement releasably carries said personal care composition; and
(b) child graphic disposed on said disposable cleaning implement said child graphic appeals to a child such that the child will interact with the implement in the typical use to which the implement is put;
**characterized in that** said personal care composition further comprises a biological extract, said biological extract being selected from the group consisting of:
(i) a sterol, said sterol preferably being selected from the group consisting of soy sterol, avocado sterols, cholesterol, sitosterol, stigmasterol, ergosterol, lanasterol, cholesterol esters, sterol esters, avocadin, lanolin, and combinations thereof;
(ii) an animal extract, said animal extract preferably being selected from the group consisting of milk protein, milk protein hydrolysate, silk protein and combinations thereof.

2. A disposable child sized cleaning implement according to Claim 1 wherein said disposable child sized cleaning implement comprises a nonwoven sheet member, said nonwoven member preferably having a laminated structure.

3. A disposable child sized cleaning implement according to Claims 1 or 2 wherein said child graphic is in the form of photographs, drawings, cartoons, symbols and combinations thereof, said child graphic preferably comprising a character graphic.

4. A disposable child sized cleaning implement according to Claim 3 wherein said child graphic is a registered graphic.

5. A disposable child sized cleaning implement according to any of the above claims, wherein said personal care composition comprises at least one of a natural fat or oil, a fatty acid, and a fatty alcohol.

6. A cleaning implement according to any of the above claims wherein said cleaning implement comprises a nonwoven mitt adapted to fit on a child's hand

7. A disposable nonwoven mitt according to Claim 6, said mitt comprising: first and second nonwoven sheet members in an overlying relationship, said members defining an interior volume for receiving said child's hand, each of said first and second nonwoven sheet members including an exterior surface, having an opposing interior surface, a top edge, a bottom edge opposing said top edge, and first and second opposed side edges, said first and second nonwoven sheet members being permanently secured to each other along the periphery of said top edge and both of said first and second opposed side edges, with said bottom edges being unsecured so as to provide a substantial access opening to said interior volume for readily inserting said child's hand therein; wherein said child graphic is disposed on at least one of said first and second sheet members.

8. An article of commerce comprising a container, said container housing at least one of said disposable child sized cleaning implements according to any of the above claims.

## Patentansprüche

1. Einwegreinigungsvorrichtung in Kindergröße, die Folgendes umfasst:
(a) eine Körperhygienezusammensetzung, wobei die Einwegreinigungsvorrichtung in Kindergröße die Körperpflegezusammensetzung in lösbarer Form trägt, und
(b) eine auf der Einwegreinigungsvorrichtung angeordnete Kindergrafik,
wobei die Kindergrafik ein Kind so anspricht, dass das Kind die Vorrichtung auf die übliche Weise, für die die Vorrichtung vorgesehen ist, verwendet,
**dadurch gekennzeichnet, dass** die Körperpflegezusammensetzung ferner einen biologischen Extrakt enthält, wobei der biologische Extrakt ausgewählt ist aus der Gruppe bestehend aus:
(I) einem Sterol, wobei das Sterol vorzugsweise ausgewählt ist aus der Gruppe bestehend aus Sojasterol, Avocadosterolen, Cholesterin, Sitosterin, Stigmasterin, Ergosterin, Lanosterin, Cholesterinestern, Sterolestern, Avocadin, Lanolin und Kombinationen davon,
(II) einem tierischen Extrakt, wobei der tierische Extrakt vorzugsweise ausgewählt ist aus der Gruppe bestehend aus Milchprotein, Milchproteinhydrolysat, Seidenprotein und Kombinationen davon.

2. Einwegreinigungsvorrichtung in Kindergröße nach Anspruch 1, wobei die Einwegreinigungsvorrichtung in Kindergröße ein Vlieslagenelement umfasst, wobei das Vlieselement vorzugsweise eine Laminatstruktur aufweist.

3. Einwegreinigungsvorrichtung in Kindergröße nach Anspruch 1 oder 2, wobei die Kindergrafik in Form von Fotografien, Zeichnungen, Cartoons, Symbolen und Kombinationen davon vorliegt, wobei die Kindergrafik vorzugsweise eine Figurgrafik umfasst.

4. Einwegreinigungsvorrichtung in Kindergröße nach Anspruch 3, wobei die Kindergrafik eine eingetragene Grafik ist.

5. Einwegreinigungsvorrichtung in Kindergröße nach einem der vorstehenden Ansprüche, wobei die Körperpflegezusammensetzung mindestens ein natürliches Fett oder Öl, eine Fettsäure und einen Fettalkohol umfasst.

6. Reinigungsvorrichtung nach einem der vorstehenden Ansprüche, wobei die Reinigungsvorrichtung einen Vlieshandschuh umfasst, der auf eine Kinderhand passt.

7. Einweg-Vlieshandschuh nach Anspruch 6, wobei der Handschuh Folgendes umfasst: ein erstes und zweites Vlieslagenelement in einer übereinander liegenden Beziehung, wobei die Elemente ein Innenvolumen zum Aufnehmen der Kinderhand begrenzen, wobei das erste und zweite Vlieslagenelement jeweils eine Außenfläche mit einer gegenüber liegenden Innenfläche, einem oberen Rand, einem dem oberen Rand gegenüber liegenden unteren Rand und einen ersten und zweiten gegenüber liegenden Seitenrand aufweist, wobei das erste und zweite Vlieslagenelement entlang des Umfangs des oberen Rands und des ersten und zweiten gegenüber liegenden Seitenrands dauerhaft aneinander befestigt sind, wobei die unteren Ränder unbefestigt sind, um eine großzügig bemessene Eingangsöffnung zum Innenvolumen zu bieten, um die Kinderhand leicht einzuführen, wobei die Kindergrafik auf mindestens einem der ersten und zweiten Lagenelemente angeordnet ist.

8. Handelsartikel, der einen Behälter umfasst, wobei der Behälter mindestens eine der Einwegreinigungsvorrichtungen in Kindergröße nach einem der vorstehenden Ansprüche enthält.

## Revendications

1. Ustensile de nettoyage jetable dimensionné pour un enfant comprenant :
(a) une composition de soin personnel, où ledit ustensile de nettoyage jetable dimensionné pour un enfant transporte de manière libérable ladite composition de soin personnel ; et
(b) une image pour enfant disposée sur ledit ustensile de nettoyage jetable, ladite image pour enfant est attirante pour un enfant de telle sorte que l'enfant interagira avec l'ustensile dans l'utilisation typique dans laquelle l'ustensile est placé ;
**caractérisé en ce que** ladite composition de soin personnel comprend en outre un extrait biologique, ledit extrait biologique étant choisi dans le groupe constitué de :
(i) un stérol, ledit stérol étant choisi de préférence dans le groupe constitué de stérol de soja, stérols d'avocat, cholestérol, sitostérol, stigmastérol, ergostérol, lanastérol, esters de cholestérol, esters de stérol, avocadine, lanoline, et leurs combinaisons ;
(ii) un extrait animal, ledit extrait animal étant choisi de préférence dans le groupe constitué de protéine de lait, hydrolysat de protéines de lait, protéine de soie et leurs combinaisons.

2. Ustensile de nettoyage jetable dimensionné pour un enfant selon la revendication 1, où ledit ustensile de nettoyage jetable dimensionné pour un enfant comprend un élément de feuille non tissé, ledit élément non tissé ayant de préférence une structure stratifiée.

3. Ustensile de nettoyage jetable dimensionné pour un enfant selon les revendications 1 ou 2, dans lequel ladite image pour enfant se présente sous la forme de photographies, dessins, dessins humoristiques, symboles et leurs combinaisons, ladite image pour enfant comprenant de préférence une image de personnage.

4. Ustensile de nettoyage jetable dimensionné pour un enfant selon la revendication 3, dans lequel ladite image pour enfant est une image déposée.

5. Ustensile de nettoyage jetable dimensionné pour un enfant selon l'une quelconque des revendications précédentes, dans lequel ladite composition de soin personnel comprend au moins un élément parmi une graisse ou huile naturelle, un acide gras et un alcool gras.

6. Ustensile de nettoyage selon l'une quelconque des revendications précédentes, où ledit ustensile de nettoyage comprend une moufle non tissée adaptée pour s'ajuster sur la main d'un enfant.

7. Moufle non tissée jetable selon la revendication 6, ladite moufle comprenant : un premier et un deuxième éléments de feuille non tissés dans un rapport de superposition de couches, lesdits éléments définissant un volume intérieur pour recevoir ladite main de l'enfant, chacun desdits premier et deuxième éléments de feuille non tissés incluant une surface extérieure, ayant une surface intérieure opposée, un bord supérieur, un bord inférieur opposé audit bord supérieur, et un premier et un deuxième bords latéraux opposés, lesdits premier et deuxième éléments de feuille non tissés fixés en permanence l'un à l'autre le long de la périphérie dudit bord supérieur et de l'un et l'autres des premier et deuxième bords latéraux opposés, lesdits bords inférieurs étant non fixés de sorte à fournir un accès substantiel s'ouvrant vers ledit volume intérieur pour insérer aisément ladite main de l'enfant dedans ; dans laquelle ladite image pour enfant est disposée sur au moins un élément parmi lesdits premier et deuxième éléments de feuille.

8. Article du commerce comprenant un récipient, ledit récipient logeant au moins un élément parmi lesdits ustensiles de nettoyage jetables dimensionnés pour un enfant selon l'une quelconque des revendications précédentes.
